# EUROPÄISCHE PATENTSCHRIFT

(11) **EP 2 758 383 B1**
(45) Veröffentlichungstag und Bekanntmachung des Hinweises auf die Patenterteilung: **09.11.2016**
(21) Anmeldenummer: 12766625.3
(22) Anmeldetag: 18.09.2012
(51) Int. Cl.: C07D 277/48, C07D 417/12, A61K 31/426, A61K 8/49, A61P 17/00, A61Q 19/02

(54) **AROMATISCHE AMIDOTHIAZOLE, SIE ENTHALTENDE KOSMETISCHE ODER DERMATOLOGISCHE ZUBEREITUNGEN UND DEREN VERWENDUNG ZUR BEKÄMPFUNG UND PROPHYLAXE UNERWÜNSCHTER PIGMENTIERUNG DER HAUT**
AROMATIC AMIDOTHIAZOLES, COSMETIC AND DERMATOLOGICAL PREPARATIONS CONTAINING THEM, AND THEIR USE FOR THE TREATMENT AND PROPHYLAXIS OF UNWANTED PIGMENTATION OF THE SKIN
AMIDOTHIAZOLES AROMATIQUES, PRÉPARATIONS COSMÉTIQUES OU DERMATOLOGIQUES LES COMPRENANT, ET LEUR UTILISATION POUR LE TRAITEMENT ET LA PRÉVENTION DE LA PIGMENTATION INDÉSIRABLE DE LA PEAU

(30) Priorität: 23.09.2011 DE 102011083271
(43) Veröffentlichungstag der Anmeldung: 30.07.2014
(73) Patentinhaber: Beiersdorf AG, 20253 Hamburg (DE)
(72) Erfinder: KOLBE, Ludger, 21255 Dohren (DE); AHLHEIT, Sabrina, 22303 Hamburg (DE); WÖHRMANN, Michael, 22851 Norderstedt (DE); SCHLÄGER, Torsten, 22297 Hamburg (DE); SCHERNER, Cathrin, 22844 Norderstedt (DE); MANN, Tobias, 22175 Hamburg (DE); GERWAT, Wolfram, 22393 Hamburg (DE)
(86) Internationale Anmeldenummer: PCT/EP2012/068373
(87) Internationale Veröffentlichungsnummer: WO 2013/041535

(56) Entgegenhaltungen:
- EP-A1- 1 649 852
- WO-A2-2011/115998
- WO-A2-2011/117034
- DATABASE CA [Online] CHEMICAL ABSTRACTS SERVICE, COLUMBUS, OHIO, US; 1998, SHRIVASTAVA, A. K. ET AL: "Syntheses of some 2-amino-4-(aryl/substituted aryl)thiazoles and their thiazolylamides as potential antifungal agents. II", XP002687441, gefunden im STN Database accession no. 1998:209541 & SHRIVASTAVA, A. K. ET AL: "Syntheses of some 2-amino-4-(aryl/substituted aryl)thiazoles and their thiazolylamides as potential antifungal agents. II", JOURNAL OF THE INSTITUTION OF CHEMISTS (INDIA) , 69(6), 167-168 CODEN: JOICA7; ISSN: 0020-3254, 1997,
- DATABASE CA [Online] CHEMICAL ABSTRACTS SERVICE, COLUMBUS, OHIO, US; 1997, SHRIVASTAVA, A. K. ET AL: "2-Amino-4-arylthiazoles and their thiazolylamides as antifungal agents. 1", XP002687442, gefunden im STN Database accession no. 1997:723423 & SHRIVASTAVA, A. K. ET AL: "2-Amino-4-arylthiazoles and their thiazolylamides as antifungal agents. 1", JOURNAL OF THE INSTITUTION OF CHEMISTS (INDIA) , 69(4), 113-115 CODEN: JOICA7; ISSN: 0020-3254, 1997,
- TRIPATHY, H. ET AL: "Search for new fungicides. I. Synthesis of some new halogenated N-thiazolyl-substituted hydroxy acid amides and their use as possible fungicides", AGRICULTURAL AND BIOLOGICAL CHEMISTRY, CODEN: ABCHA6; ISSN: 0002-1369, Bd. 37, Nr. 6, 1973, Seiten 1375-1383, XP002687443,
- HUSAIN I ET AL: "SEARCH FOR POTENT ANTHELMINTICS-PART XIII 2-(3,5-SUBSTITUTED SALICYLAMIDO/CINNAMIDO)-4,5-SUBSTITUTED THIAZOLES", JOURNAL OF THE INDIAN CHEMICAL SOCIETY, THE INDIAN CHEMICAL SOCIETY, CALCUTTA; IN, Bd. 56, Nr. 9, 1. September 1979 (1979-09-01), Seite 917/918, XP008063181, ISSN: 0019-4522
- DATABASE REGISTRY [Online] CHEMICAL ABSTRACTS SERVICE, COLUMBUS, OHIO, US; 22. September 2010 (2010-09-22), chemical library; supplier: sigma-aldrich: XP002687444, Database accession no. 1242267-72-6
- GERMANAS ET AL: "Discovery of small-molecule inhibitors of tyrosinase", BIOORGANIC & MEDICINAL CHEMISTRY LETTERS, PERGAMON, ELSEVIER SCIENCE, GB, Bd. 17, Nr. 24, 12. Oktober 2007 (2007-10-12), Seiten 6871-6875, XP022339589, ISSN: 0960-894X, DOI: 10.1016/J.BMCL.2007.10.014 in der Anmeldung erwähnt

## Beschreibung

Die vorliegende Erfindung betrifft neue Aromatische Amidothiazole, kosmetische oder dermatologische Zubereitungen mit einem Gehalt an einem oder mehreren solcher Aromatischer Amidothiazole und die Verwendung solcher Aromatischer Amidothiazole bzw. Zubereitungen, solche Aromatische Amidothiazole enthaltend, zur Bekämpfung und Prophylaxe unerwünschter Pigmentierung der Haut.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozeß der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden daß für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides),* genetische Disposition, Fehlpigmentierung der Haut bei der Wundheilung bzw. - vernarbung (postinflammatorische Hyperpigmentierung) oder der Hautalterung (z.B. *Lentigines seniles).*

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté-Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen werden derartige postinflammatorische Fehlpigmentierung durch Einwirkung von Sonnenlicht (UV-Licht) noch verstärkt, ohne daß es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte "Triformula" entwickelt, die eine Kombination aus 0.1% Tretinoin, 5.0% Hydrochinon, 0.1% Dexamethason darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Weiterhin sind diverse andere Substanzen bekannt, für die eine hautaufhellende Wirksamkeit beschrieben wird. U.a. zu nennen sind hier Hexadecen-1,16-dicarbonsäure, Kojic-Säure und Derivate, Arbutin, Ascorbinsäure und Derivate, Flavonoide, Ellagsäure und Derivate, Tranexamsäure und verschiedene Resorcinol-Derivate, wie z.B. 4-n-Butylresorcin, 4-n-Hexylresorcin und 4-(1-phenylethyl)benzen-1,3-diol.

J.M. Ready beschreibt in einer Publikation (Bioorganic & Medicinal Chemistry Letter 17 (2007) 6871-6875 die Wirkung von u.a. substituierten Thiazol-Derivaten zur Inhibition der Mushroom tyrosinase.

In der Patentanmeldung der Firma Shiseido (WO 2009099195) werden substituierte Thiazolamine bzw. Hydrothiazolamine zur Hautaufhellung beschrieben.

Die im oben genannten Stand der Technik beschriebenen Substanzen weisen sich durch eine moderate Wirksamkeit und/oder eine schlechte galenische Stabilität aus.

Augenringe können ebenfalls als Folgen einer Pigmentierungsstörung entstehen, wobei sie ferner auch als Reaktion auf allgemeinen Stress, wie z.B. wenig Schlaf oder schlicht durch Überanstrengung der Augen erscheinen. Bei jüngeren Menschen verschwinden die Symptome nach ausreichender Nachtruhe wieder, über längere Zeiträume jedoch kann der Zustand chronisch und für die betroffenen Personen sehr störend werden. Auch gegen solche Hauterscheinungen mangelt es an genügend erfolgversprechenden Wirkstoffen und Behandlungsmöglichkeiten.

Ziel der nachfolgenden Erfindung war es also, dem nachteiligen Stand der Technik Abhilfe zu verschaffen.

Die Lösung der Aufgaben, der der Erfindung zugrunde liegen, besteht in Aromatische Amidothiazole, dadurch gekennzeichnet, daß sie eine der folgenden Strukturen aufweisen: 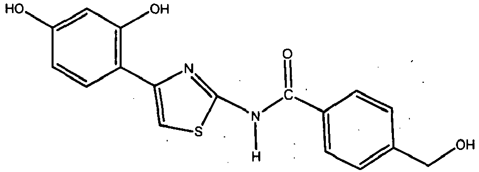 N-(4-(2,4-dihydroxyphenyl)thiazol-2-yl)-4-(hydroxymethyl)benzamid 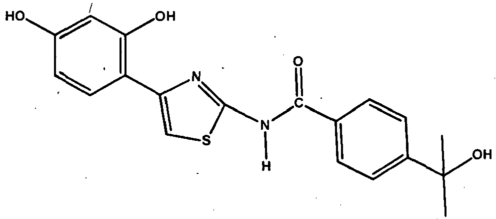 N-(4-(2,4-dihydroxyphenyl)thiazol-2-yl)-4-(2-hydroxypropan-2-yl)benzamid

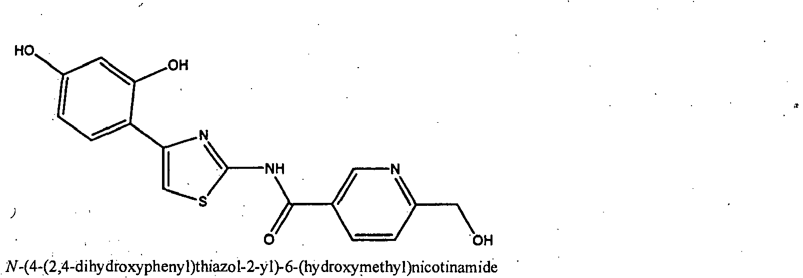

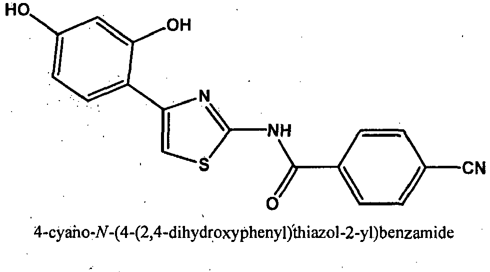

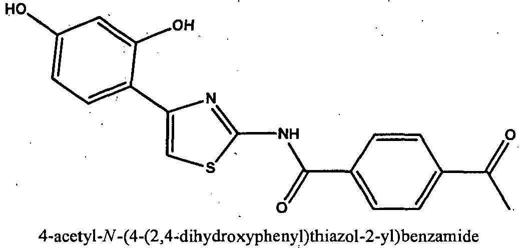

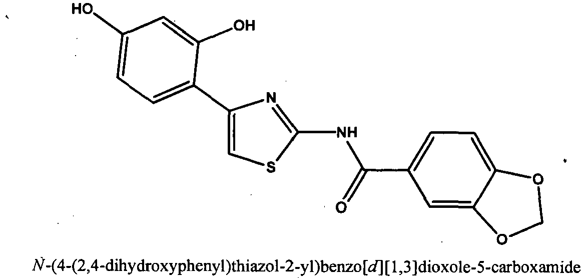

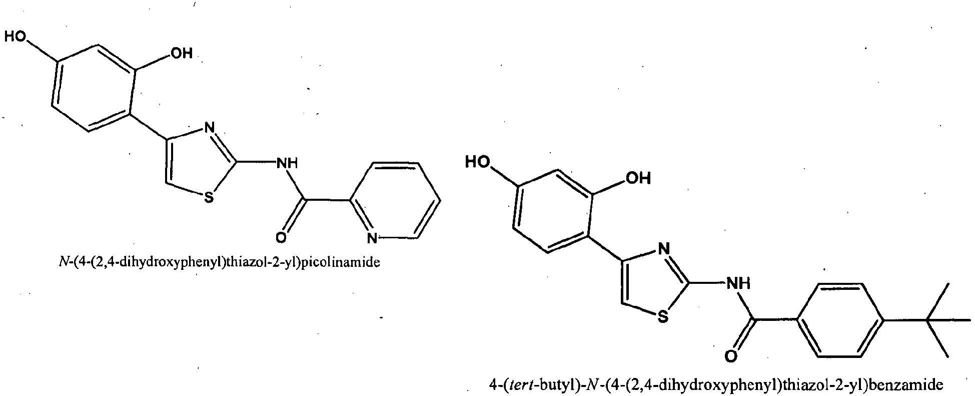

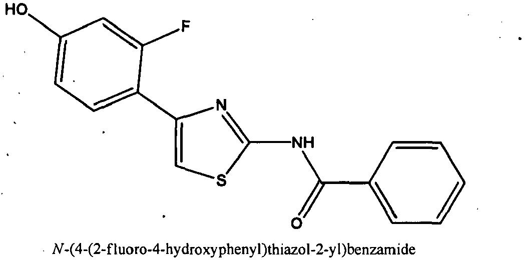

Die genannten Thiazole können sowohl als freie Base wie auch als Salz vorliegen: z.B. als Fluorid, Chlorid, Bromid, lodid, Sulfat, Carbonat, Ascorbat, Acetat oder Phoshat. Im Besonderen als Halogensalze, wie z.B. Chlorid und Bromid.

Weiterhin besteht eine vorteilhafte Verwirklichung der vorliegenden Erfindung in kosmetischen oder dermatologischen Zubereitungen mit einem wirksamen Gehalt an einem oder mehreren vorbenannten Aromatischen Amidothiazolen.

Erfindungsgemäß ist ferner die Verwendung der vorgenannten Aromatischen Amidothiazole zur Behandlung und/oder Prophylaxe unerwünschter Hautpigmentierung.

Dabei können Behandlung und/oder Prophylaxe unerwünschter Hautpigmentierung sowohl im kosmetischen wie im pharmazeutischen Rahmen erfolgen.

Dabei, wird die pharmazeutische (oder dermatologische) Behandlung in erster Linie bei krankhaften Hautzuständen verstanden, wogegen die kosmetische Behandlung und/oder Prophylaxe unerwünschter Hautpigmentierung in erster Linie die gesunde Haut betrifft.

Überraschenderweise konnte gezeigt werden, daß die erfindungsgemäßen Aromatischen Amidothiazole im Vergleich zu den entsprechenden Aromatischen Aminothiazolen eine höhere galenische Stabilität aufweisen und/oder eine gesteigerte Wirksamkeit.

### Siehe Tabelle 1.

### Methodenbeschreibung der Wirksamkeitsuntersuchungen:

Die Wirksamkeit der Thiazole wurde mit einem Enzymtest belegt, in der Umsatz von L-DOPA zu L-Dopachinon durch eine humane Tyrosinase gemessen wurde. Bei dieser literaturbekannten Methode (Winder, A.J. and Harris, H., New assays for the tyrosine hydroxylase and dopa oxidase activities of tyrosinase. Eur. J. Biochem. (1991), 198, 317-26) wird das Reaktionsprodukt L-Dopaquinone mit MBTH (3-methyl-2-benzothiazoline hydrazone) zu einer pinkfarbenen Substanz umgesetzt, deren Zunahme über die Zeit durch Absorption bei 490 nm gemessen wird. In Tabelle ein sind Wirksamkeitsdaten für einige der beanspruchten Substanzen beispielhaft dargestellt. Daraus lässt sich schließen, dass die erfindungsgemä-ßen Substanzen äußerst effektive pigmentierungs-inhibierende Substanzen sind.

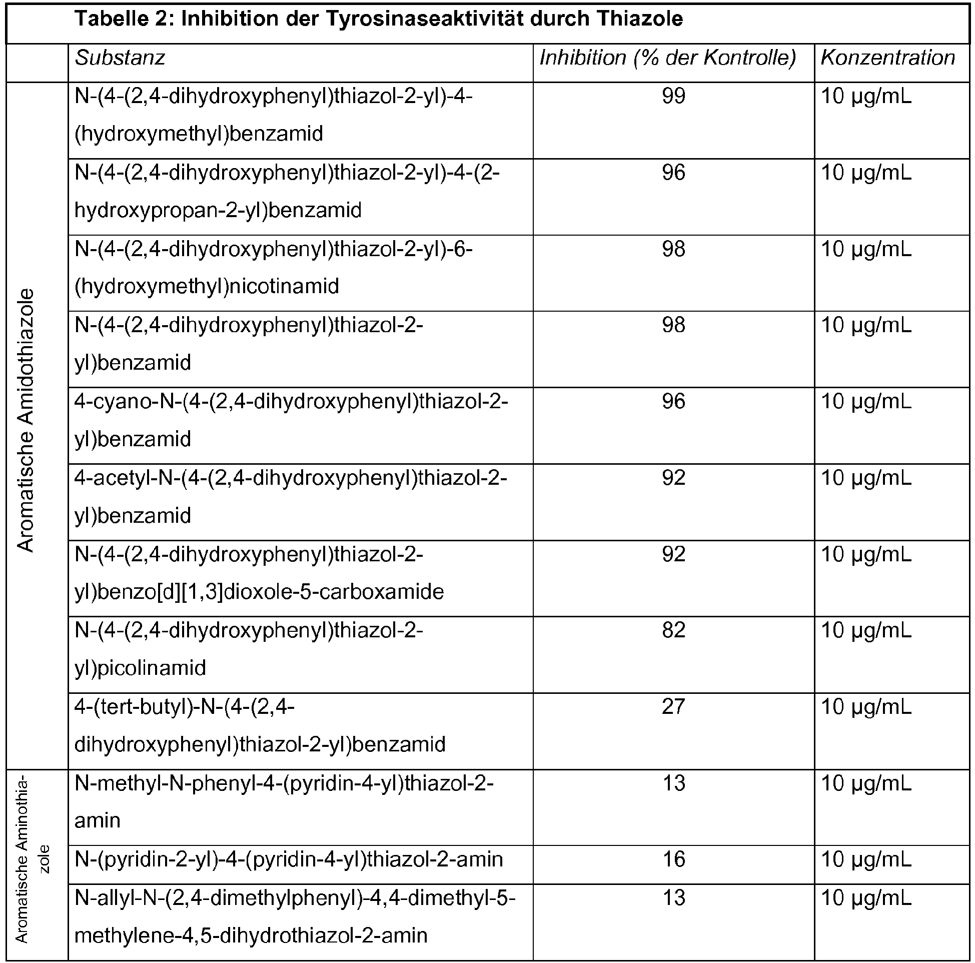

### Synthesevorschriften exemplarisch ausgewählter Aromatischer Amidothiazole:

### 2-Brom-2',4'- bis-methoxycarbonyloxy-acetophenon:

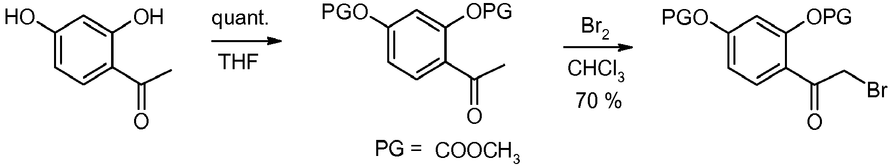

Mitchell, David; Doecke, Christopher W.; Hay, Lynne A.; Koenig, Thomas M.; Wirth, David D. Tetrahedron Letters, 1995

Eine Lösung von 60g (369 mmol) 2,4-Dihydroxyacetophenon and 186 ml Triethylamin in 900 ml Tetrahydrofuran wurde auf 0°C gekühlt und 93 ml Chlorameisensäuremethylester in 400 ml Tetrahydrofuran langsam zugetropft. Es bildet sich ein weißer Niederschlag. Nach 3 Stunden Rühren bei Raumtemperatur ist die Reaktion abgeschlossen (DC-Kontrolle). Der Niederschlag wurde abgesaugt und mit reichlich Tetrahydrofuran gewaschen. Das Filtrat wurde zur Trockne einrotiert, in Ethylacetat aufgenommen mit 1N HCl und NaCl-Lösung (sat.) gewaschen und über Magnesiumsulfat getrocknet, vom Magnesiumsulfat filtriert und das Ethylacetat am Rotationsverdampfer eingeengt. Es wurden 105 g von 2,4-Bis-methoxycarbonyloxy-acetophenon erhalten. ¹H NMR (DMSO-D₆): 8.05 (d, 1H), 7.38 (d, 1H), 7.36 (s, 1H), 3.86 (d, 6H). Das Produkt wurde ohne weitere Reinigung eingesetzt. Zu der Lösung von 105 g 2,4-Bis-methoxycarbonyloxy-acetophenon in Chloroform (1000 ml) wurden 63g (392 mmol) Brom in 450 ml Chloroform innerhalb von 3 h zugetropft. Danach wurde die Reaktion noch 15 min. bei Raumtemperatur gerührt, Das Lösungsmittel wurde einrotiert. Der Rückstand wurde in Ethylacetat/n-Hexan verrührt, der entstandene Niederschlag wurde abgesaugt. Umkristallisation aus Ethylacetat/n-Hexan lieferten 100 g 2-Brom-2',4'-bis-methoxycarbonyloxy-acetophenon. ¹H NMR (DMSO-D₆): 8.11 (d, 1H), 7.42 (m, 2H), 4. 87 (s, 2H), 3.87 (s, 3H), 3.85 (s, 3H) ppm; m.p. 73-74°C.

### N-(4-(2,4-dihydroxyphenyl)thiazol-2-yl)-4-(hydroxymethyl)benzamid:

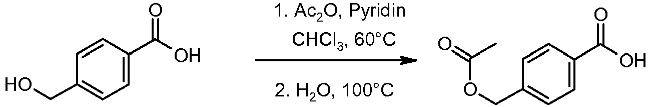

Durchführung nach Literatur. Ausbeute: 88 %
**BANYU Pharmaceutical Co., Ltd.,** EP2072519A1, 2009

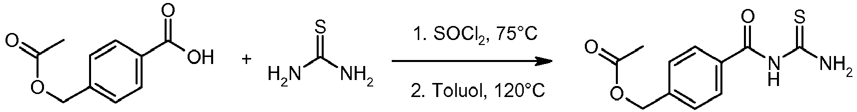

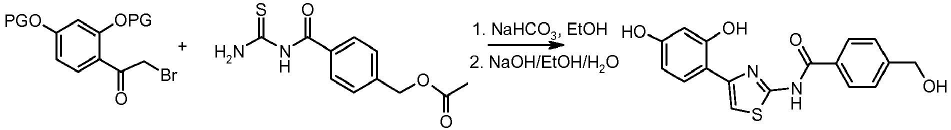

73.06 g (380 mmol) 4-Acetoxymethylbenzoesäure wurden in 350 ml Thionylchlorid 2 h unter Rückfluss erhitzt. Nach Entfernen des überschüssigen Thionylchlorids im Vakuum wurde der Rückstand in 1000 ml Toluol aufgenommen und 57 g (750 mmol) Thioharnstoff zugegeben. Die Reaktionslösung wurde 3 Stunden unter Rückfluss gekocht und anschließend wurde das Lösungsmittel im Vakuum entfernt. Der Feststoff wurde mit 500 ml Methanol und 500 ml Ethylacetat aufgekocht und heiß filtriert. Ausbeute: 55 g. ¹H NMR (DMSO-D₆): 11.25 (bs, 1H), 9.85 (bs, 1H), 9.56 (bs, 1H), 7.93 (d, 2H), 7.47 (d, 2H), 5.15 (s, 2H), 2.10 (s, 3H) ppm.

74.3 g (218 mmol) 2-Brom-2',4'- bis-methoxycarbonyloxy-acetophenon wurden mit 55 g (218 mmol) Thioharnstoff und 28 g (327 mmol) NaHCO₃ in 1000 ml Ethanol unter Rückfluss für 0.5h gekocht. Die Reaktionslösung wurde abgekühlt und mit 70 g (1.74 mol) NaOH in 300 ml Wasser versetzt. Nach 2 h Rühren bei 60°C wurde die Reaktionslösung in 500 ml Wasser aufgenommen und mit 2N HCl auf pH=6 eingestellt. Der entstandene Niederschlag wurde abfiltriert und aus Ethanol/Wasser umkristallisiert. Es wurden 46 g Thiazol erhalten. ¹H NMR (DMSO-D₆): 12.62 (bs, 1H), 11.08 (bs, 1H), 9.50 (bs, 1H), 8.08 (d, 2H), 7.70 (d, 1H), 7.51 (d, 2H), 7.48 (s, 1 H), 6.32 (m, 2H), 5.38 (t, 1 H), 4.61 (d, 2H) ppm. m.p.: 251-254°C.

### N-(4-(2,4-dihydroxyphenyl)thiazol-2-yl)-4-(2-hydroxypropan-2-yl)benzamid:

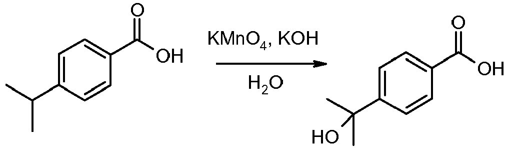

Durchführung nach Literatur. Ausbeute: 37 %
US-20070032484 A1 Seite 48

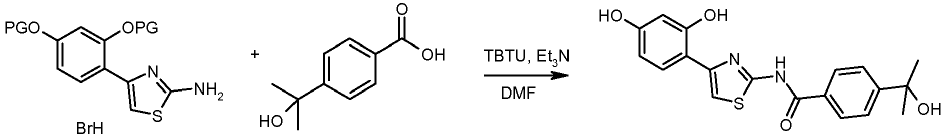

4.9 g (12.2 mmol) Thiazol-hydrobromid und 2.2 g (12.2 mmol) 4-(Hydroxy-1-methyl-ethyl)benzoesäure wurden mit 4.3 g (13.4 mmol) TBTU sowie 6.17g (61 mmol) Triethylamin in 50 ml DMF bei Raumtemperatur über Nacht gerührt. Dimethylformamid wurde am Rotationsverdampfer abgezogen und das Reaktionsgemisch in 150 ml Ethanol aufgenommen, 2.5 g (61 mmol) NaOH in 15 ml Wasser zugegeben und eine Stunde bei Raumtemperatur gerührt. Danach wurden 30 ml Wasser zugefügt und mit 1 N HCl auf pH= 5 eingestellt. Das Lösungsmittel wurde im Vakuum entfernt und die Reinigung des Rückstandes erfolgte mittels Säulenchromatographie an Kieselgel 60 mit Chloroform/Methanol/NH₃ 9/1/0.1. Ausbeute: 1.3 g. ¹H NMR (DMSO-D₆): 12.59 (br, 1H), 11.06 (br, 1H), 9.49 (br, 1 H), 8.05 (d, 2H), 7.70 (d, 1 H), 7.65 (d, 2H), 7.48 (s, 1 H), 6.30 (m, 2H), 5.20 (s, 1 H), 1.46 (s, 6H) ppm; m.p.: 152-154°C.

### N-(4-(2,4-dihydroxyphenyl)thiazol-2-yl)-6-(hydroxymethyl)nicotinamid:

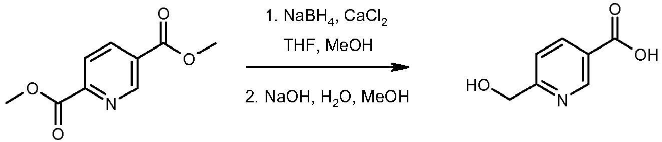

Durchführung nach Literatur. Ausbeute: 50%
1. Sirtris Pharmaceuticals Inc., WO2010/5654 A1, 2010
2. Pfizer Inc., US2007/270438 A1, 2007

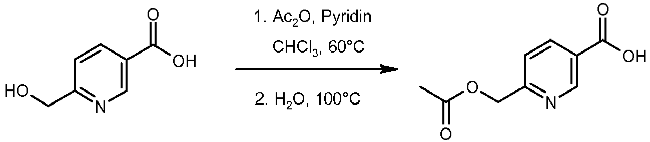

Durchführung analog Literatur.

BANYU Pharmaceutical Co., Ltd., EP2072519 A1, 2009

Ausbeute: 91%, ¹H NMR (DMSO-D₆): 13.27 (bs, 1 H), 9.03 (d, 1 H), 8.29 (dd, 1 H), 7.54 (d, 1 H), 5.22 (s, 2H), 2.15 (s, 3H) ppm;

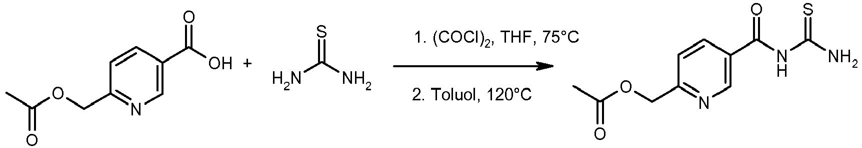

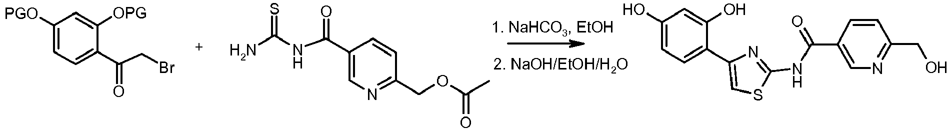

5.0 g (26 mmol) 6-Acetoxymethylnicotinsäure wurden in 100 ml THF gelöst, 3.7 g (31 mmol) Oxalylchlorid wurden zugegeben und anschließend 15 min unter Rückfluss erhitzt. 3.9 g (51 mmol) Thioharnstoff wurden zugegeben und dann die Reaktionslösung 3 Stunden unter Rückfluss erhitzt. Das Lösungsmittel wurde im Vakuum entfernt und die Reinigung erfolgte mittels Säulenchromatographie an Kieselgel mit Chloroform/Methanol 9/1. Ausbeute: 1.2 g. ¹H NMR (DMSO-D₆): 11.54 (s, 1 H), 9.77 (bs, 1 H), 9.62 (bs, 1 H), 8.98 (d, 1 H), 8.28 (dd, 1 H), 7.52 (d, 1H), 5.21 (s, 2H), 2.15 (s, 3H) ppm;
1.6 g (4.7 mmol) 2-Brom-2',4'- bis-methoxycarbonyloxy-acetophenon wurden mit 1.2 g (4.7 mmol) N-(6-Acetoxymethylnicotinoyl)thioharnstoff und 0.6 g (7.1 mmol) NaHCO₃ in 20 ml Ethanol unter Rückfluss für 0.5h gekocht. Die Reaktionslösung wurde abgekühlt und mit 2.0 g (50 mmol) NaOH in 10 ml Wasser versetzt. Nach 2 h Rühren bei 60°C wurde die Reaktionslösung in 50 ml Wasser aufgenommen und mit 2N HCl auf pH=6 eingestellt. Die Reaktionsmischung wurde im Vakuum auf ca. 20 ml eingeengt und der entstandene Niederschlag wurde abfiltriert. Die Reinigung erfolgte durch präparative HPLC (Kieselgel, RP 18, Wasser/Acetonitril/TFA 50/50/0.1) und anschließende Fällung als Hydrochlorid. Es wurden 0.3 g Thiazol erhalten. ¹H NMR (DMSO-D₆): 13.00 (bs, 1H), 9.22 (bs, 1H), 8.61 (d, 1H), 7.79 (d, 1 H), 7.71 (d, 1 H), 7.53 (s, 1 H), 6.37 (d, 1 H), 6.33 (dd, 2H), 5.80 (bs, 4H), 4.79 (s, 2H) ppm. m.p.: > 202°C Zers.

### 4-cyano-N-(4-(2,4-dihydroxyphenyl)thiazol-2-yl)benzamid:

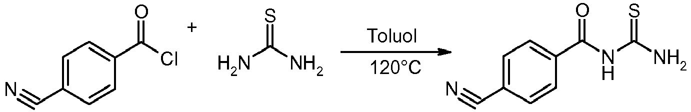

10.6 g (139 mmol) Thioharnstoff wurden in Toluol (100 ml) vorgelegt und 11.5 g (69.5 mmol) 4-Cyanobenzoylchlorid zugetropft. Die Reaktionslösung wurde 4 Stunden unter Rückfluss gekocht. Das Lösungsmittel wurde einrotiert und der Rückstand wurde dreimal mit Acetonitril ausgekocht und heiß filtriert. Die aus der abgekühlten Lösung ausgefallenen Kristalle wurden abgesaugt und getrocknet. Ausbeute: 7.7 g. ¹H NMR (DMSO-D₆): 11.54 (bs, 1H), 9.74 (bs, 1 H), 9.64 (bs, 1 H), 8.05 (d, 2H), 8.02 (d, 2H) ppm.

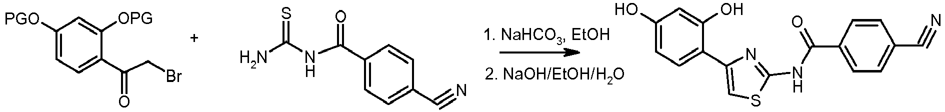

13.02 g (37 mmol) 2-Brom-2',4'- bis-methoxycarbonyloxy-acetophenon wurden mit 7.7 g (37 mmol) 4-Cyano benzoylthioharnstoff und 4.72 g (56 mmol) NaHCO₃ in 100 ml Ethanol unter Rückfluss für 40 Min. gekocht. Die Reaktionslösung wurde abgekühlt und mit 6.0 g (148 mmol) NaOH in 100 ml Wasser versetzt. Nach 30 Min. Rühren bei Raumtemperatur wurde die Reaktionslösung mit 50 ml Wasser versetzt und mit 2N HCl auf pH = 4 eingestellt und am Rotationsverdampfer das Ethanol abgezogen. Der entstandene Niederschlag wurde abfiltriert und mit Ethanol zweimal ausgekocht und heiß filtriert. Aus der abgekühlten Lösung wurden 6.3 g Thiazol erhalten. ¹H NMR (DMSO-D₆): 12.92 (bs, 1H), 10.93 (bs, 1H), 9.52 (bs, 1H), 8.24 (d, 2H), 8.06 (d, 2H), 7.71 (d, 1H), 7.70 (s, 1H), 6.33 (m, 2H) ppm. m.p.: 301-303°C.

### 4-(tert-butyl)-N-(4-(2,4-dihydroxyphenyl)thiazol-2-yl)benzamid:

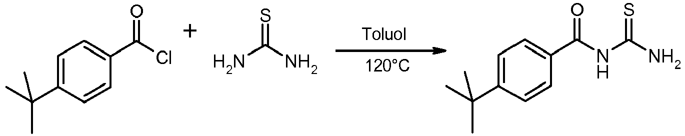

7.74 g (102 mmol) Thioharnstoff wurden in Toluol (75 ml) vorgelegt und 10 g (50.8 mmol) p-tert-Butyl-benzoylchlorid zugetropft. Die Reaktionslösung wurde 4 Stunden unter Rückfluss gekocht und abgekühlt. Die ausgefallenen leicht gelblichen Kristalle wurden aus Ethanol zweimal umkristallisiert. Ausbeute: 4.1 g. ¹H NMR (DMSO-D₆): 11.11 (bs, 1H), 9.88 (bs, 1H), 9.53 (bs, 1 H), 7.90 (d, 2H), 7.53 (d, 2H), 1.30 (s, 9H) ppm.

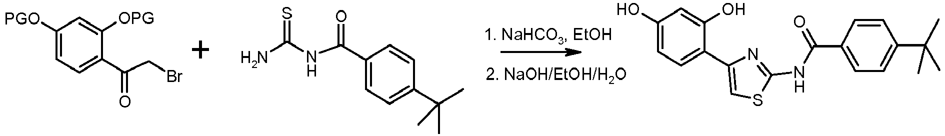

4.71 g (13.6 mmol) 2-Brom-2',4'- bis-methoxycarbonyloxy-acetophenon wurden mit 3.21 g (13.6 mmol) p-tert-Butyl-benzoylthioharnstoff und 1.72 g (20.4 mmol) NaHCO₃ in 45 ml Ethanol unter Rückfluss für 0.5h gekocht. Die Reaktionslösung wurde abgekühlt und mit 2.0g (50 mmol) NaOH in 15 ml Wasser versetzt. Nach 30 Min. Rühren bei Raumtemperatur wurde die Reaktionslösung mit 30 ml Wasser aufgenommen und mit 2N HCl auf pH = 6 eingestellt. Der entstandene Niederschlag wurde abfiltriert und aus Ethanol/Wasser zweimal umkristallisiert. Es wurden 3.2 g Thiazol erhalten. ¹H NMR (DMSO-D₆): 12.59 (bs, 1H), 11.06 (bs, 1 H), 9.50 (bs, 1 H), 8.06 (d, 2H), 7.70 (d, 1 H), 7.59 (d, 2H), 7.48 (s, 1 H), 6.32 (m, 2H), 1.33 (s, 9H) ppm. m.p.: 228-229°C.

Kosmetische oder dermatologische Zubereitungen mit einem Gehalt an Aromatischen Amidothiazolen bzw. deren Verwendung zur Behandlung und/oder Prophylaxe unerwünschter Hautpigmentierung, sind ebenfalls vorteilhafte Verkörperungen der vorliegenden Erfindung.

Vorteilhaft ist es insbesondere, wenn solche Zubereitungen 0,000001 bis 10 Gew.-%, insbesondere 0,0001 bis 3 Gew.-%, ganz besonders 0,001 bis 1 Gew.-% an einem oder mehreren der erfindungsgemäß verwendeten Aromatischen Amidothiazolen enthalten, bezogen auf das Gesamtgewicht der Zubereitung.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindungsgemäß vorteilhaft, die erfindungsgemäß verwendeten Substanzen und/oder deren Derivate in verkapselter Form darzureichen, z.B. in Kollagenmatrices und anderen üblichen Verkapselungsmaterialien, z.B. als Celluloseverkapselungen, in Gelatine oder liposomal verkapselt.

Es ist auch möglich und vorteilhaft im Sinne der vorliegenden Erfindung, die erfindungsgemäß verwendeten Substanzen und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Siliconderivate.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Feuchthaltemittel wie z.B. Propylenglycol, Panthenol oder Hyaluronsäure sowie insbesondere ein oder mehrere Verdickungsmittel, welches oder welche vorteilhaft gewählt werden können aus der Gruppe Siliciumdioxid, Aluminiumsilikate, Hydroxypropylmethylcellulose, besonders vorteilhaft ein Polyacrylat wie beispielsweise Carbopole Typ 980, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Propyleglycol, und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

### Rezepturbeispiele

### O/W -Emulsionen

| **Rezepturbeispiel** | **1** | **2** | **3** | **4** |
|---|---|---|---|---|
| **Chemische/INCI-Bezeichnung** | **Gew.-%** | **Gew.-%** | **Gew.-%** | **Gew.-%** |
| Stearinsäure | 2,50 | 2,00 | 2,00 | 2,50 |
| Glyceryl Stearat | 1,00 | 1,00 | 1,00 | 1,00 |
| C12-15 Alkyl Benzoat | 3,00 | 5,00 | 3,00 | 2,00 |
| Caprylsäure/Caprinsäure Triglycerid | 2,50 | 2,50 | 2,00 | 2,50 |
| Isopropyl Palmitat | 2,00 | - | - | 2,00 |
| Cetylstearylalkohol | 3,00 | - | 2,00 | 3,00 |
| Cetyl Alkohol | - | 2,00 | - | - |
| Stearyl Alkohol | - | 2,00 | 1,00 | - |
| Cyclomethicon | 1,00 | 1,00 | 0,50 | - |
| Dicaprylyl Carbonat | 2,00 | 2,00 | 2,00 | 2,00 |
| Dimethicon | 1,00 | - | 0,50 | 1,00 |
| Glycerin | 5,00 | 7,00 | 5,00 | 9,00 |
| Methylparaben | 0,20 | - | - | - |
| Phenoxyethanol | 0,40 | 0,50 | 0,50 | 0,40 |
| Propylparaben | 0,10 | - | - | 0,10 |
| 1,2-Hexandiol | - | - | 0,10 | 0,10 |
| Ethylhexylglycerin | - | - | 0,20 | - |
| Methylisothiazolinon | - | 0,05 | - | - |
| Butylenglykol | - | - | 2,0 | - |
| Carbomer | 0,15 | 0,10 | 0,15 | 0,10 |
| Carrageenan | 0,10 | - | 0,10 | - |
| Xanthan Gummi | - | - | 0,10 | - |
| Acrylates/C10-30 Alkyl Acrylate Cross-polymer | - | 0,10 | - | 0,10 |
| Trinatrium EDTA | 0,20 | 0,20 | 0,20 | 0,20 |
| Tapioka Stärke | 1,50 | 1,00 | - | |
| Nylon-12 (1,8-Diazacyclotetradecan-2,7-dion Homopolymer) | - | 0,20 | - | 0,50 |
| Polymethylsilsesquioxane | - | 1,00 | 1,00 | - |
| Aluminum Stärke Octenylsuccinat | - | - | 1,00 | - |
| Distärkephosphat | 1,00 | - | - | 1,00 |
| Butyl Methoxydibenzoylmethan | 1,00 | 2,00 | 1,00 | 1,00 |
| Phenylbenzimidazole Sulfonsäure | - | 1,00 | 2,00 | - |
| Octocrylen | - | 2,00 | 1,00 | - |
| Ethylhexyl Salicylat | 1,00 | - | - | 1,00 |
| N-(4-(2,4-dihydroxyphenyl)thiazol-2-yl)-4-(hydroxymethyl)benzamid | 0,20 | 0.10 | 0.05 | 0.30 |
| N-(4-(2,4-dihydroxyphenyl)thiazol-2-yl)-4-(2-hydroxypropan-2-yl)benzamid | 0.01 | 0.25 | 0.15 | 0.10 |
| N-(4-(2,4-dihydroxyphenyl)thiazol-2-yl)-6-(hydroxymethyl)nicotinamid | 0.25 | 0.15 | 0.30 | 0.35 |
| N-(4-(2,4-dihydroxyphenyl)thiazol-2-yl)benzamid | 0.10 | 0.10 | 0.15 | 0.20 |
| Natriumhydroxid | q.s. | q.s. | q.s. | q.s. |
| Hydroxyisohexyl 3-Cyclohexencarboxaldehyd | 0,10 | - | - | 0,05 |
| Citronellol | 0,05 | 0,10 | - | 0,05 |
| Linalool | - | 0,05 | 0,10 | - |
| Parfüm | 0,30 | 0,20 | 0,20 | 0,20 |
| Natriumhydroxid | q.s. | q.s. | q.s. | q.s. |
| Wasser | ad 100 | ad 100 | ad 100 | ad 100 |

| **Rezepturbeispiel** | **5** | **6** | **7** | **8** |
|---|---|---|---|---|
| **Chemische Bezeichnung** | **Gew.-%** | **Gew.-%** | **Gew.-%** | **Gew.-%** |
| Glyceryl Stearate Citrat | 2,00 | 1,50 | 2,00 | 2,00 |
| Behenyl Alkohol | 1,50 | 1,00 | 1,00 | 1,00 |
| C12-15 Alkyl Benzoat | 2,00 | 2,50 | 2,00 | 2,50 |
| Caprylsäure/Caprinsäure Triglycerid | 2,00 | 2,00 | 2,50 | 2,50 |
| Cetyl Alkohol | 2,00 | 2,00 | - | 2,00 |
| Cetylstearylalkohol | - | - | 2,00 | - |
| Cyclomethicon | 1,00 | 1,00 | 2,00 | 2,00 |
| Dicaprylyl Carbonat | - | 2,00 | 2,50 | 2,50 |
| Paraffinum Liquidum (Mineralöl) | - | - | 0,50 | - |
| Octyldodecanol | - | 2,00 | - | - |
| Dimethicon | 0,50 | 1,00 | 1,00 | - |
| Glycerin | 3,00 | 5,00 | 7,00 | 9,00 |
| Methylparaben | 0,20 | 0,15 | - | - |
| Phenoxyethanol | 0,40 | 0,60 | 0,50 | 0,50 |
| Propylparaben | 0,10 | - | - | - |
| Methylisothiazolinon | - | - | 0,05 | - |
| Piroctone Olamine | - | - | - | 0,15 |
| Glyceryl Caprylat | - | - | - | 0,20 |
| Carbomer | 0,20 | - | 0,15 | 0,15 |
| Natrium Polyacrylat | - | 0,40 | - | - |
| Xanthan Gummi | 0,10 | - | 0,10 | - |
| Acrylates/C10-30 Alkyl Acrylate Cross-polymer | - | 0,10 | - | 0,10 |
| Tapioka Stärke | 0,50 | - | 0,50 | - |
| Nylon-12 (1,8-Diazacyclotetradecan-2,7-dion Homopolymer) | 1,00 | - | - | 1,00 |
| Polymethylsilsesquioxane | - | 1,00 | 1,00 | - |
| Aluminum Stärke Octenylsuccinat | - | 1,00 | - | 1,00 |
| N-(4-(2,4-dihydroxyphenyl)thiazol-2-yl)benzo[d][1,3]dioxole-5-carboxamid | 0.25 | 0.15 | 0.30 | 0.35 |
| N-(4-(2,4-dihydroxyphenyl)thiazol-2-yl)picolinamid | 0.10 | 0.10 | 0.15 | 0.20 |
| 4-(tert-butyl)-N-(4-(2,4-dihydroxyphenyl)thiazol-2-yl)benzamid | 0.01 | 0.25 | 0.15 | 0.10 |
| N-(4-(2-fluoro-4-hydroxyphenyl)thiazol-2-yl)benzamid | 0,20 | 0.10 | 0.05 | 0.30 |
| Glycyrrhiza Inflata Root Extrakt | 0,03 | 0,05 | 0,05 | 0,03 |
| Titandioxid | - | 1,00 | - | - |
| Octocrylene | - | 1,00 | - | 2,00 |
| Bis-Ethylhexyloxyphenol Methoxyphenyl Triazin | - | 1,00 | 1,00 | - |
| 2-Ethylhexyl Methoxycinnamat | - | 1,00 | 2,00 | 2,00 |
| Homosalat (3,3,5-Trimethyl-cyclohexylsalicylat) | - | - | 1,00 | 1,00 |
| Natriumhydroxid | q.s. | q.s. | q.s. | q.s. |
| Trinatrium EDTA | 0,15 | - | 0,15 | - |
| 1-(1,2,3,4,5,6,7,8-octahydro-2,3,8,8,-tetramethyl-2-naphthyl)ethan-1-one | 0,1 | - | q.s. | q.s. |
| Geraniol | - | 0,05 | - | - |
| Hexylcinnamal | - | - | 0,05 | - |
| Parfüm | 0,10 | 0,20 | 0,30 | 0,20 |
| Wasser | ad 100 | ad 100 | ad 100 | ad 100 |

| **Rezepturbeispiele** | **9** | **10** | **11** | **12** |
|---|---|---|---|---|
| **Chemische Bezeichnung** | **Gew.-%** | **Gew.-%** | **Gew.-%** | **Gew.-%** |
| Polyglyceryl-3 Methylglucose Distearat | 2,00 | 2,50 | 2,50 | 2,50 |
| Sorbitan Stearate | 1,50 | 3,00 | 1,50 | 3,00 |
| C12-15 Alkyl Benzoat | 2,50 | 2,50 | 2,50 | 2,50 |
| Caprylsäure/Caprinsäure Triglyceride | 2,50 | 2,50 | 2,50 | 2,50 |
| Stearyl Alkohol | 1,00 | 1,50 | 1,00 | 1,50 |
| Cyclomethicon | 3,00 | 1,00 | 2,00 | 1,00 |
| Isopropyl Myristat | - | 2,50 | 2,00 | 2,50 |
| Isopropyl Palmitat | 2,00 | - | 1,00 | - |
| Dimethicon | - | 1,00 | - | 1,00 |
| Glycerin | 5,00 | 7,50 | 3,00 | 7,50 |
| Butyrospermum Parkii Butter | 2,00 | - | - | - |
| Methylparaben | 0,20 | 0,20 | - | 0,10 |
| Phenoxyethanol | 0,40 | 0,40 | 0,40 | 0,40 |
| Propylparaben | 0,10 | - | - | - |
| Benzethonium chlorid | - | - | 0,10 | - |
| Caprylyl Glycol | - | 0,20 | - | - |
| Ethylhexylglycerin | - | 0,20 | - | 0,2 |
| Carbomer | 0,15 | 0,10 | 0,15 | 0,10 |
| Ammonium Acryloyldimethyltaurate/VP Copolymer | - | 0,20 | - | 0,20 |
| Carrageenan | 0,10 | - | 0,15 | - |
| Trinatrium EDTA | - | 1,00 | - | 1,00 |
| Tapioka Stärke | - | 1,00 | 1,00 | - |
| Distärke Phosphat | - | 1,00 | - | 1,00 |
| Acrylonitrile-methacrylonitrile-methyl-methacrylate Copolymer + Isopentane + Magnesium Hydroxide | - | - | 1,00 | 1,00 |
| N-(4-(2,4-dihydroxyphenyl)thiazol-2-yl)-6-(hydroxymethyl)nicotinamid | 0.01 | 0.25 | 0.15 | 0.10 |
| N-(4-(2,4-dihydroxyphenyl)thiazol-2-yl)benzamid | 0,20 | 0.10 | 0.05 | 0.30 |
| 4-cyano-N-(4-(2,4-dihydroxyphenyl)thiazol-2-yl)benzamid | 0.25 | 0.15 | 0.30 | 0.35 |
| 4-acetyl-N-(4-(2,4-dihydroxyphenyl)thiazol-2-yl)benzamid | 0.10 | 0.10 | 0.15 | 0.20 |
| Diethylamino Hydroxybenzoyl Hexyl Benzoate | - | - | 1,00 | - |
| Ethylhexyl Methoxycinnamat | - | 1,00 | - | 2,00 |
| Butyl Methoxydibenzoylmethan | - | 2,00 | - | 2,00 |
| Octocrylen | - | 1,00 | 2,00 | 1,00 |
| Titandioxid | - | - | 1,00 | - |
| Natriumhydroxid | q.s. | q.s. | q.s. | q.s. |
| Ubiquinone | 0,10 | - | - | - |
| Natrium Metabisulfit | - | 0,15 | - | - |
| BHT (tert-Butylhydroxytoluol) | - | - | 0,05 | - |
| Linalyacetat | 0,05 | - | - | - |
| Hexylsalicylat | - | 0,05 | - | - |
| Benzylsalicylat | - | - | 0,01 | - |
| Parfüm | q.s. | q.s. | q.s. | q.s. |
| Wasser | ad 100 | ad 100 | ad 100 | ad 100 |

| **Rezepturbeispiele** | **13** | **14** | **15** | **16** |
|---|---|---|---|---|
| **Chemische Bezeichnung** | **Gew.-%** | **Gew.-%** | **Gew.-%** | **Gew.-%** |
| PEG-40 Stearate | 0,80 | 1,00 | 1,00 | 1,00 |
| Glyceryl Stearate | 2,50 | 3,00 | 3,00 | 3,00 |
| C12-15 Alkyl Benzoate | 2,00 | 2,50 | 2,00 | 2,00 |
| Caprylsäure/Caprinsäure Triglycerid | 2,00 | 2,50 | 2,50 | 2,00 |
| Cetylstearylalkohol | 3,00 | 3,00 | 3,00 | 3,00 |
| Cyclomethicon | 2,00 | 2,00 | 2,00 | 2,00 |
| Dicaprylyl Carbonat | - | 2,00 | 2,50 | 2,50 |
| Octyldodecanol | 1,00 | - | - | 1,50 |
| Butyrospermum Parkii Butter | 2,00 | - | - | - |
| Octyldodecyl Myristat | 1,00 | - | 1,50 | 1,00 |
| Dimethicon | 1,00 | 1,00 | 1,00 | 1,00 |
| Glycerin | 7,50 | 5,00 | 9,0 | 7,50 |
| Methylparaben | 0,20 | - | 0,10 | - |
| Phenoxyethanol | 0,40 | 0,50 | 0,40 | 0,40 |
| Propylparaben | 0,10 | - | - | - |
| Glyceryl Caprylat | - | 0,25 | - | - |
| Pentylenglykol | - | 0,50 | - | - |
| Butylenglykol | - | - | 3,00 | - |
| Carbomer | 0,15 | 0,10 | 0,10 | 0,15 |
| Natrium Polyacrylat | - | 0,20 | 0,20 | - |
| Xanthan Gummi | 0,10 | - | - | - |
| Acrylates/C10-30 Alkyl Acrylate Crosspolymer | - | - | - | 0,1 |
| Trinatrium EDTA + Wasser (20%ige wässrige Lösung) | - | 1,00 | 1,00 | 1,00 |
| Tapioca Stärke | - | 1,00 | 1,00 | 1,00 |
| Distärke Phosphat | - | 1,00 | 1,00 | 1,00 |
| Aluminum Stärke Octenylsuccinat | 2,00 | - | - | - |
| Acrylonitrile-methacrylonitrile-methyl-methacrylate Copolymer + Isopentane + Magnesium Hydroxide | 1,00 | - | - | - |
| N-(4-(2,4-dihydroxyphenyl)thiazol-2-yl)benzo[d][1,3]dioxole-5-carboxamid | 0,10 | 0,15 | 0,10 | 0,01 |
| N-(4-(2,4-dihydroxyphenyl)thiazol-2-yl)picolinamid | 0,20 | 0,10 | 0,05 | 0,20 |
| 4-(tert-butyl)-N-(4-(2,4-dihydroxyphenyl)thiazol-2-yl)benzamid | 0,30 | 0.15 | 0,10 | 0,10 |
| Ethylhexyl Methoxycinnamat | - | 1,00 | 1,00 | 2,00 |
| Diethylamino Hydroxybenzoyl Hexyl Benzoat | - | 1,00 | - | 1,00 |
| Titandioxid | - | - | 1,00 | - |
| Homosalate (3,3,5-Trimethyl-cyclohexylsalicylat) | - | - | 2,00 | - |
| Phenylbenzimidazole Sulfonsäure | | | 1,00 | |
| Natrium Metabisulfit | 0,10 | - | - | - |
| Natriumhydroxid | q.s. | q.s. | q.s. | q.s. |
| 3-Methyl-5-phenyl-1-pentanol | 0,10 | - | - | - |
| Coumarin | - | 0,05 | - | - |
| Ethyllinalool | - | - | 0,10 | - |
| Ascorbylpalmitat | 0,10 | - | - | - |
| Parfüm | q.s. | q.s. | q.s. | q.s. |
| Wasser | ad 100 | ad 100 | ad 100 | ad 100 |

| **Rezepturbeispiele** | **17** | **18** | **19** | **20** |
|---|---|---|---|---|
| **Chemische Bezeichnung** | **Gew.-%** | **Gew.-%** | **Gew.-%** | **Gew.-%** |
| Glyceryl Stearate Citrat | 2,00 | 2,00 | 2,00 | 2,00 |
| Isopropyl Palmitat | 3,00 | 2,00 | 3,00 | 1,00 |
| Cetylstearylalkohol | 4,00 | 3,00 | 3,00 | - |
| Cetylalkohol | - | - | - | 4,00 |
| Caprylsäure/Caprinsäure Triglycerid | 3,00 | 2,50 | 2,00 | 3,00 |
| C12-15 Alkyl Benzoat | 3,00 | 2,50 | 2,00 | 2,00 |
| Cyclomethicon | 1,00 | - | 1,00 | - |
| Dicaprylyl Carbonat | - | - | 2,50 | - |
| Dimethicon | - | 0,50 | - | - |
| Octyldodecyl Myristat | - | 1,00 | - | - |
| Glycerin | 4,00 | 6,00 | 5,00 | 6,00 |
| Methylparaben | 0,20 | - | 0,10 | - |
| Phenoxyethanol | 0,40 | 0,40 | 0,40 | 0,40 |
| Piroctone Olamine | - | - | - | 0,10 |
| Ethylhexylglycerin | - | 0,30 | - | - |
| Glyceryl Caprylate | - | 0,30 | - | - |
| 2-Methyl-1,3-propandiol | - | 2,00 | - | 2,00 |
| Carbomer | 0,20 | 0,10 | 0,15 | - |
| Natrium Polyacrylate | - | 0,40 | - | - |
| Xanthan Gummi | 0,10 | - | - | 0,15 |
| Acrylates/C10-30 Alkyl Acrylate Crosspolymer | - | - | 0,10 | 0,20 |
| Acrylonitrile-methacrylonitrile-methyl-methacrylate Copolymer + Isopentane + Magnesium Hydroxide | 0,50 | - | 0,50 | - |
| Aluminum Stärke Octenylsuccinat | - | 1,00 | - | 1,00 |
| Methyl Methacrylate Crosspolymer | 1,00 | | - | 1,00 |
| N-(4-(2,4-dihydroxyphenyl)thiazol-2-yl)-4-(hydroxymethyl)benzamid | 0,25 | 0,30 | 0,01 | 0,05 |
| N-(4-(2,4-dihydroxyphenyl)thiazol-2-yl)-4-(2-hydroxypropan-2-yl)benzamid | 0,10 | 0,25 | 0,15 | 0,10 |
| N-(4-(2,4-dihydroxyphenyl)thiazol-2-yl)-6-(hydroxymethyl)nicotinamid | 0,15 | 0,15 | 0,01 | 0,06 |
| N-(4-(2,4-dihydroxyphenyl)thiazol-2-yl)benzamid | 0,20 | 0,30 | 0,10 | 0,05 |
| Bis-Ethylhexyloxyphenol Methoxyphenyl Triazines | - | | 1,00 | |
| Titandioxid | - | 1,00 | - | 1,00 |
| Octocrylene | - | 1,00 | 1,00 | 1,00 |
| Butyl Methoxydibenzoylmethan | - | 1,00 | - | 1,00 |
| Ethylhexyl Salicylat | - | - | 1,00 | - |
| Citronellol | 0,05 | - | 0,05 | - |
| Coumarin | 0,05 | 0,05 | - | 0,05 |
| Triethylcitrat | - | - | 0,05 | 0,05 |
| Natriumhydroxid | q.s. | q.s. | q.s. | q.s. |
| Wasser | ad 100 | ad 100 | ad 100 | ad 100 |

| **Rezepturbeispiele** | **21** | **22** | **23** | **24** |
|---|---|---|---|---|
| Chemische **Bezeichnung** | **Gew.-%** | **Gew.-%** | **Gew.-%** | **Gew.-%** |
| Sucrose Polystearate + Hydrogynated Polvisobutene | 1,00 | 1,00 | 2,00 | 2,00 |
| Natrium Stearoyl Glutamat | 0,20 | 0,20 | 0,30 | 0,30 |
| C12-15 Alkyl Benzoat | 1,50 | 1,50 | - | - |
| Cetyl Alkohol | 0,50 | 0,50 | - | - |
| Cyclomethicon | 10,00 | 10,00 | 5,00 | 5,00 |
| Dimethicon | 3,00 | 3,00 | 2,50 | 2,50 |
| Glycerin | 7,50 | 7,50 | 5,00 | 5,00 |
| Isopropyl Stearat | 1,00 | 1,00 | 2,00 | 2,00 |
| Paraffinum Liquidum (Mineralöl) | 3,00 | 3,00 | 1,00 | 1,00 |
| Methylparaben | 0,10 | - | - | 0,10 |
| Ethylhexylglycerin | - | - | 0,30 | 0,10 |
| Propylparaben | 0,10 | - | - | - |
| Methylisothiazolinon | - | 0,05 | - | - |
| Phenoxyethanol | 0,40 | 0,50 | 0,40 | 0,40 |
| N-(4-(2,4-dihydroxyphenyl)thiazol-2-yl)-4-(2-hydroxypropan-2-yl)benzamid | 0,10 | 0,10 | 0,05 | 0,05 |
| N-(4-(2,4-dihydroxyphenyl)thiazol-2-yl)-6-(hydroxymethyl)nicotinamid | 0,10 | 0,25 | 0,15 | 0,10 |
| N-(4-(2,4-dihydroxyphenyl)thiazol-2-yl)benzamid | 0,15 | 0,15 | 0,01 | 0,06 |
| 4-cyano-N-(4-(2,4-dihydroxyphenyl)thiazol-2-yl)benzamid | 0,20 | 0,30 | 0,10 | 0,05 |
| Ethylhexyl Methoxycinnamat | 3,00 | 2,00 | 3,00 | 3,00 |
| Butyl Methoxydibenzoylmethan | 2,00 | 2,00 | 1,00 | 1,00 |
| Phenylbenzimidazole Sulfonsäure | - | 1,50 | - | 1,00 |
| Butylenglykol | - | - | 3,00 | |
| Polymethylsilsesquioxan | - | - | 1,00 | 1,00 |
| Nylon-12 (1,8-Diazacyclotetradecan-2,7-dion Homopolymer) | - | 1,00 | 1,00 | - |
| Distärke Phosphat | - | 1,00 | - | 1,00 |
| Methyl Methacrylate Crosspolymer | 1,00 | - | - | - |
| Aluminum Starch Octenylsuccinat | 1,00 | - | - | - |
| Ammonium Acryloyldimethyltaurate/VP Copolymer | - | - | 0,25 | 0,25 |
| Xanthan Gummi | 0,10 | - | - | 0,10 |
| Acrylates/C10-30 Alkyl Acrylate Crosspolymer | 0,25 | 0,10 | - | - |
| Carbomer | - | 0,10 | 0,10 | - |
| Hexylcinnamal | 0,05 | 0,10 | - | 0,10 |
| 1-(1,2,3,4,5,6,7,8-octahydro-2,3,8,8,-tetramethyl-2-naphthyl)ethan-1-one | - | 0,10 | 0,10 | - |
| Linalool | - | - | 0,05 | 0,05 |
| Parfüm | 0,20 | 0,20 | 0,20 | 0,20 |
| Natriumhydroxid | q.s. | q.s. | q.s. | q.s. |
| Wasser | ad 100 | ad 100 | ad 100 | ad 100 |

| **Rezepturbeispiele** | **25** | **26** | **27** | **28** |
|---|---|---|---|---|
| **Chemische Bezeichnung** | **Gew.-%** | **Gew.-%** | **Gew.-%** | **Gew.-%** |
| Natrium Cetearyl Sulfat | 0,15 | 0,15 | 0,15 | 0,15 |
| Glyceryl Stearate SE | 2,00 | 2,00 | 1,50 | 1,50 |
| C12-15 Alkyl Benzoat | 2,50 | 2,50 | 2,50 | 2,50 |
| Octyldodecanol | 1,00 | 1,00 | - | - |
| Caprylsäure/Caprinsäure Triglycerid | 2,00 | 2,00 | 2,00 | 2,00 |
| Cetylstearylalkohol | 2,00 | 2,00 | 3,00 | 1,00 |
| Cyclomethicon | 1,50 | 1,50 | 2,50 | 2,50 |
| Dimethicon | 0,50 | 0,50 | 0,50 | 0,50 |
| Glycerin | 5,00 | 5,00 | 7,50 | 7,50 |
| Isopropyl Stearat | 3,00 | 3,00 | 2,00 | 2,00 |
| Paraffinum Liquidum (Mineralöl) | 2,00 | 2,00 | 1,00 | 1,00 |
| Methylisothiazolinon | - | - | - | 0,05 |
| Phenoxyethanol | 0,40 | 0,50 | 0,40 | 0,30 |
| Methylparaben | 0,15 | - | - | - |
| Propylparaben | 0,10 | - | - | - |
| Piroctone Olamine | - | 0,15 | - | - |
| Benzethonium chlorid | - | - | 0,10 | - |
| N-(4-(2,4-dihydroxyphenyl)thiazol-2-yl)benzamid | 0,10 | 0,10 | 0,05 | 0,05 |
| 4-cyano-N-(4-(2,4-dihydroxyphenyl)thiazol-2-yl)benzamid | 0,25 | 0,30 | 0,01 | 0,05 |
| 4-acetyl-N-(4-(2,4-dihydroxyphenyl)thiazol-2-yl)benzamid | 0,10 | 0,25 | 0,15 | 0,10 |
| N-(4-(2,4-dihydroxyphenyl)thiazol-2-yl)benzo[d][1,3]dioxole-5-carboxamid | 0,15 | 0,15 | 0,01 | 0,06 |
| Ethylhexyl Methoxycinnamat | 3,00 | 3,00 | 5,00 | 5,00 |
| Butyl Methoxydibenzoylmethan | 1,00 | 1,00 | 2,00 | 2,00 |
| Pentylenglykol | - | 1,00 | 1,00 | - |
| Butylenglycol | 1,00 | 1,50 | 3,00 | 3,00 |
| 2-Methyl-1,3-propandiol | - | - | - | - |
| 1,2-Hexandiol | - | - | - | 1,00 |
| Nylon-12 (1,8-Diazacyclotetradecan-2,7-dion Homopolymer) | 1,00 | 1,00 | 1,00 | 1,00 |
| Carbomer | - | - | 0,10 | 0,15 |
| Ammonium Acryloyldimethyltaurate/VP Copolymer | 0,20 | - | - | - |
| Chondrus Crispus | 0,10 | 0,10 | - | - |
| Xanthan Gummi | - | - | 0,10 | - |
| Acrylates/C10-30 Alkyl Acrylate Crosspolymer | - | 0,20 | 0,10 | 0,10 |
| Coumarin | 0,10 | - | 0,05 | 0,05 |
| Hydroxyisohexyl3-Cyclohexencarboxaldehyde | 0,05 | 0,05 | 0,05 | 0,10 |
| 1-(1,2,3,4,5,6,7,8-octahydro-2,3,8,8,-tetramethyl-2-naphthyl)ethan-1-one | - | 0,05 | 0,10 | - |
| Parfüm | 0,20 | 0,30 | 0,40 | 0,20 |
| Natriumhydroxid | q.s. | q.s. | q.s. | q.s. |
| Wasser | ad 100 | ad 100 | ad 100 | ad 100 |

| **Rezepturbeispiele** | **29** | **30** | **31** | **32** |
|---|---|---|---|---|
| **Chemische/INCI-Bezeichnung** | **Gew.-%** | **Gew.-%** | **Gew.-%** | **Gew.-%** |
| Natrium Cetearyl Sulfat | 0,15 | 0,15 | 0,20 | 0,20 |
| Glyceryl Stearate, selbstemulgierend | 2,00 | 2,00 | 1,50 | 1,50 |
| C12-15 Alkyl Benzoat | 2,00 | 2,00 | 2,00 | 2,00 |
| Octyldodecanol | 1,00 | 1,00 | - | - |
| Caprylsäure/Caprinsäure Triglycerid | 2,00 | 2,00 | 2,00 | 2,00 |
| Cetylstearylalkohol | 2,00 | 2,00 | 1,00 | 1,00 |
| Cyclomethicon | 1,00 | 1,00 | 2,00 | 2,00 |
| Dimethicon | 0,50 | 0,50 | 1,00 | 1,00 |
| Glycerin | 5,00 | 5,00 | 7,50 | 7,50 |
| Isopropyl Palmitat | 2,50 | 2,50 | 2,00 | 2,00 |
| DMDM Hydantoin | 0,05 | 0,05 | 0,05 | 0,05 |
| Phenoxyethanol | 0,35 | 0,25 | 0,30 | 0,30 |
| Ethanol | - | - | 3,00 | 2,00 |
| Pentylenglykol | 1,00 | - | 1,00 | 1,50 |
| 4-Acetyl-N-(4-(2,4-dihydroxyphenyl)thiazol-2-yl)benzamid | 0,10 | 0,10 | 0,05 | 0,05 |
| N-(4-(2,4-dihydroxyphenyl)thiazol-2-yl)benzo[d][1,3]dioxole-5-carboxamid | 0,25 | 0,30 | 0,01 | 0,05 |
| N-(4-(2,4-dihydroxyphenyl)thiazol-2-yl)picolinamid | 0,10 | 0,25 | 0,15 | 0,10 |
| Carbomer | 0,20 | 0,20 | 0,20 | 0,20 |
| Carrageenan | 0,10 | 0,10 | - | - |
| Xanthan Gummi | - | - | 0,20 | 0,20 |
| Acrylates/C10-30 Alkyl Acrylate Crosspolymer | - | - | - | 0,15 |
| Natrium Polyacrylat | - | 0,20 | - | - |
| Diethylhexyl 2,6-Naphthalat | - | - | 1,00 | - |
| Phenylbenzimidazole Sulfonsäure | - | 1,00 | - | 2,00 |
| Titandioxid | - | - | 1,00 | - |
| Diethylamino Hydroxybenzoyl Hexyl Benzoat | - | - | 1,00 | 1,00 |
| Octocrylene | - | 4,00 | 2,00 | 3,00 |
| 3,3,5-Trimethylcyclohexylsalicylat | | 1,00 | - | - |
| Distärkepjosphat | - | 1,00 | 1,00 | - |
| Methyl Methacrylate Crosspolymer | 1,00 | - | - | 1,00 |
| Polymethylsilsesquioxane | - | - | 1,00 | 1,00 |
| Acrylonitrile-methacrylonitrile-methyl-methacrylate Copolymer + Isopentane + Magnesium Hydroxid | 1,00 | 1,00 | - | - |
| 1-(1,2,3,4,5,6,7,8-octahydro-2,3,8,8,-tetramethyl-2-naphthyl)ethan-1-one | - | 0,10 | 0,10 | 0,05 |
| Hydroxyisohexyl 3-Cyclohexencarboxaldehyde | 0,05 | 0,05 | 0,10 | - |
| Linalylacetat | 0,10 | - | 0,05 | 0,05 |
| Parfüm | 0,15 | 0,15 | 0,30 | 0,30 |
| Natriumhydroxid | q.s. | q.s. | q.s. | q.s. |
| Wasser | ad 100 | ad 100 | ad 100 | ad 100 |

| **Rezepturbeispiele** | **33** | **34** | **35** | **36** |
|---|---|---|---|---|
| **Chemische**/**INCI**-**Bezeichnung** | **Gew.-%** | **Gew.-%** | **Gew.-%** | **Gew.-%** |
| Natrium Cetearyl Sulfat | 0,15 | 0,15 | 0,15 | 0,15 |
| Glyceryl Stearate, selbstemulgierend | 1,00 | 1,00 | 1,00 | 1,00 |
| C12-15 Alkyl Benzoat | 2,00 | 2,50 | 2,00 | 2,00 |
| Isopropyl Palmitat | 3,50 | 3,00 | 2,50 | 3,50 |
| Dimethicon | 1,00 | 1,00 | 1,00 | 1,0 |
| Cetylstearylalkohol | 1,00 | 1,00 | 1,00 | 1,00 |
| Octyldodecyl Myristate | - | - | - | 1,00 |
| Butyrospermum Parkii Butter | - | - | 1,00 | - |
| Glycerin | 7,00 | 3,00 | 9,00 | 5,00 |
| Carbomer | 0,10 | 0,15 | 0,10 | 0,10 |
| Acrylates/C10-30 Alkyl Acrylate Crosspolymer | 0,15 | 0,10 | 0,10 | 0,15 |
| Xanthan Gummi | 0,15 | 0,15 | 0,15 | 0,15 |
| Phenylbenzimidazole Sulfonsäure | 1,00 | 1,00 | - | 1,00 |
| Butyl Methoxydibenzoylmethan | 1,50 | 1,50 | 1,50 | 1,50 |
| Ethylhexyl Salicylat | 2,00 | 2,50 | 2,50 | 2,50 |
| Octocrylene | 1,50 | 1,50 | 2,50 | 1,50 |
| Titanium Dioxide + Trimethoxycaprylylsilan | 1,00 | - | 1,00 | - |
| Aluminum Stärke Octenylsuccinat | - | 1,00 | - | 0,50 |
| Methyl Methacrylate Crosspolymer | 0,50 | - | 0,50 | - |
| Nylon-12 (1,8-Diazacyclotetradecan-2,7-dion Homopolymer) | 0,50 | - | 1,00 | - |
| Tapioka Stärke | 0,50 | 0,50 | - | 1,00 |
| Phenoxyethanol | 0,50 | 0,50 | 0,50 | 0,40 |
| Ethylhexylglycerin | 0,25 | - | 0,25 | - |
| 1,2-Hexandiol | - | 1,00 | - | 3,00 |
| Caprylyl Glycol | - | 0,30 | 0,30 | - |
| 2-Methyl-1,2-propandiol | 2,00 | 2,00 | 2,00 | - |
| N-(4-(2,4-dihydroxyphenyl)thiazol-2-yl)-4-(2-hydroxypropan-2-yl)benzamid | 0,10 | 0,10 | 0,05 | 0,05 |
| N-(4-(2,4-dihydroxyphenyl)thiazol-2-yl)-6-(hydroxymethyl)nicotinamid | 0,25 | 0,30 | 0,01 | 0,05 |
| N-(4-(2,4-dihydroxyphenyl)thiazol-2-yl)benzamid | 0,10 | 0,25 | 0,15 | 0,10 |
| Ethyllinalool | 0,05 | - | 0,05 | - |
| 3-Methyl-5-phenyl-1-pentanol | - | 0,05 | - | 0,05 |
| Geraniol | 0,05 | - | 0,05 | - |
| Natriumhydroxid | q.s. | q.s. | q.s. | q.s. |
| Wasser | ad 100 | ad 100 | ad 100 | ad 100 |

| **Rezepturbeispiele** | **37** | **38** | **39** | **40** |
|---|---|---|---|---|
| **Chemische/INCI**-**Bezeichnung** | **Gew.-%** | **Gew.-%** | **Gew.-%** | **Gew.-%** |
| Polyglycerly-10 Stearat | 0,20 | 0,20 | 0,20 | 0,20 |
| Glyceryl Stearat | 3,00 | 0,50 | 0,50 | 0,50 |
| C12-15 Alkyl Benzoat | 4,00 | 2,00 | 1,50 | 2,50 |
| Isopropyl Palmitat | 4,00 | 1,00 | 2,00 | 2,50 |
| Caprylsäure/Caprinsäure Triglycerid | 4,00 | 3,00 | 2,00 | 2,50 |
| Hydrogenated Coco-Glycerides | 3,00 | - | - | 2,00 |
| Butyrospermum Parkii Butter | 3,00 | - | 2,50 | - |
| Cetylstearylalkohol | 5,00 | 3,50 | 4,00 | 3,00 |
| Paraffinum Liquidum (Mineralöl) | - | - | - | 1,00 |
| Glycerin | 5,00 | 3,00 | 7,00 | 9,00 |
| Acrylates/C10-30 Alkyl Acrylate Crosspolymer | 0,30 | 0,20 | 0,15 | 0,20 |
| Methylisothiazolinon | 0,05 | - | - | 0,05 |
| Phenoxyethanol | 0,50 | 0,40 | 0,40 | 0,40 |
| Carbomer | 0,10 | 0,15 | 0,10 | 0,10 |
| Methylparaben | - | 0,10 | 0,10 | - |
| Propylparaben | - | 0,10 | - | - |
| Nylon-12 (1,8-Diazacyclotetradecan-2,7-dion Homopolymer) | 1,00 | 0,50 | - | - |
| Polymethylsilsesquioxane | - | 1,00 | 0,50 | - |
| Methyl Methacrylate Crosspolymer | - | - | 1,00 | 0,50 |
| Tapioka Stärke | 0,50 | - | - | 0,50 |
| N-(4-(2,4-dihydroxyphenyl)thiazol-2-yl)benzo[d][1,3]dioxole-5-carboxamid | 0,25 | 0,30 | 0,01 | 0,05 |
| N-(4-(2,4-dihydroxyphenyl)thiazol-2-yl)picolinamid | 0,10 | 0,25 | 0,15 | 0,10 |
| 4-(tert-butyl)-N-(4-(2,4-dihydroxyphenyl)thiazol-2-yl)benzamid | 0,15 | 0,15 | 0,01 | 0,06 |
| N-(4-(2-fluoro-4-hydroxyphenyl)thiazol-2-yl)benzamid | 0,20 | 0,30 | 0,10 | 0,05 |
| Ethanol | 3,00 | - | 2,00 | - |
| Geraniol | 0,05 | 0,05 | - | - |
| Benzylsalicylat | - | 0,05 | 0,05 | - |
| Ethyllinalool | - | - | 0,05 | 0,05 |
| Parfüm | 0,20 | 0,15 | 0,30 | 0,30 |
| Natriumhydroxid | q.s. | q.s. | q.s. | q.s. |
| Wasser | ad 100 | ad 100 | ad 100 | ad 100 |

| **Rezepturbeispiele** | **41** | **42** | **43** | **44** |
|---|---|---|---|---|
| **Chemische**/**INCI**-**Bezeichnung** | **Gew.-%** | **Gew.-%** | **Gew.-%** | **Gew.-%** |
| Polyglycerly-10 Stearat | 0,20 | 0,20 | 0,15 | 0,15 |
| C12-15 Alkyl Benzoat | 2,50 | 2,50 | 2,00 | 3,00 |
| Isopropyl Palmitat | 2,50 | 2,50 | 2,00 | 2,00 |
| Caprvlsäure/Caprinsäure Triglycerid | 2,00 | 2,50 | 1,00 | 2,00 |
| Glyceryl Stearat | 1,00 | 1,00 | 0,50 | 0,50 |
| Octyldodecanol | 0,50 | - | - | 1,00 |
| Cyclomethicon | - | - | 0,50 | 0,50 |
| Butyl Methoxydibenzoylmethan | - | 2,00 | 2,00 | - |
| Octocrylene | - | 2,00 | 3,00 | 2,00 |
| Ethylhexyl Salicylat | - | 1,00 | 1,00 | - |
| Phenylbenzimidazole Sulfonsäure | - | 1,00 | - | 1,50 |
| Titandioxid | - | 1,00 | - | 1,00 |
| 3,3,5-Trimethylcyclohexylsalicylat | - | - | 1,00 | 1,00 |
| Glycerin | 9,00 | 5,00 | 7,00 | 7,00 |
| Tapioka Stärke | 1,00 | 1,00 | - | - |
| Acrylonitrile-methacrylonitrile-methyl-methacrylate Copolymer + Isopentane + Magnesium Hydroxide | - | 1,00 | 0,50 | - |
| Aluminum Stärke Octenylsuccinat | - | - | 1,00 | 1,00 |
| Distärkephosphat | - | - | - | 1,00 |
| Methylisothiazolinon | 0,05 | 0,05 | - | - |
| Phenoxyethanol | 0,50 | 0,50 | 0,40 | 0,40 |
| Benzethoniumchlorid | - | - | 0,10 | - |
| Ethylhexylqlycerin | - | - | 0,10 | - |
| Methylparaben | - | - | - | 0,20 |
| Carbomer | 0,25 | 0,20 | 0,20 | 0,20 |
| Acrylates/C10-30 Alkyl Acrylate Crosspolymer | 0,20 | - | - | 0,15 |
| Ammonium Acryloyldimethyltaurate/VP Copolymer | - | 0,25 | - | - |
| Natrium Polyacrylat | - | - | 0,30 | - |
| Xanthan Gummi | - | - | - | 0,15 |
| N-(4-(2,4-dihydroxyphenyl)thiazol-2-yl)-4-(hydroxymethyl)benzamid | 0.25 | 0.15 | 0.30 | 0.35 |
| N-(4-(2,4-dihydroxyphenyl)thiazol-2-yl)-4-(2-hydroxypropan-2-yl)benzamid | 0.10 | 0.10 | 0.15 | 0.20 |
| N-(4-(2,4-dihydroxyphenyl)thiazol-2-yl)-6-(hydroxymethyl)nicotinamid | 0.01 | 0.25 | 0.15 | 0.10 |
| N-(4-(2,4-dihydroxyphenyl)thiazol-2-yl)benzamid | 0,20 | 0.10 | 0.05 | 0.30 |
| Ethanol | 3,00 | 3,00 | - | - |
| Butylenglykol | - | - | 2,00 | 2,00 |
| Coumarin | - | 0,05 | 0,05 | - |
| Hexylcinnamal | 0,05 | 0,05 | - | 0,05 |
| Hexylsalicylat | - | - | 0,05 | 0,05 |
| Parfüm | 0,15 | 0,20 | 0,25 | 0,30 |
| Natriumhydroxid | q.s. | q.s. | q.s. | q.s. |
| Wasser | ad 100 | ad 100 | ad 100 | ad 100 |

| **Rezepturbeispiele** | **45** | **46** | **47** | **48** |
|---|---|---|---|---|
| **Chemische/INCI-Bezeichnung** | **Gew.-%** | **Gew.-%** | **Gew.-%** | **Gew.-%** |
| Kalium Cetylphosphat | 0,20 | 0,20 | 0,20 | 0,20 |
| Dicaprylyl Carbonat | - | 1,00 | - | - |
| C12-15 Alkyl Benzoat | 2,50 | 2,00 | 1,00 | 3,00 |
| Isopropyl Palmitat | 2,50 | 2,00 | 3,00 | 1,00 |
| Caprylsäure/Caprinsäure Triglycerid | 2,50 | 2,00 | 1,50 | 2,00 |
| Cera Microcristallina | - | - | - | 0,50 |
| Cylcomethicon | 0,25 | - | 0,50 | 0,50 |
| Diethylhexyl 2,6-Naphthalat | - | 0,50 | - | 1,00 |
| Diethylamino Hydroxybenzoyl Hexyl Benzoat | - | 1,00 | - | 1,00 |
| Ethylhexyl Salicylat | - | - | 2,00 | 1,00 |
| Octocrylen | - | - | 3,00 | 2,00 |
| Glycerin | 5,00 | 7,00 | 9,00 | 7,00 |
| Acrylates/C10-30 Alkyl Acrylate Crosspolymer | 0,10 | 0,30 | - | 0,10 |
| Natrium Polyacrylat | 0,30 | - | - | - |
| Carbomer | - | 0,10 | 0,15 | 0,15 |
| Ammonium Acryloyldimethyltaurate/VP Copolymer | - | - | 0,25 | - |
| Chondrus Crispus Extrakt (Carrageenan) | - | - | - | 0,10 |
| Methylisothiazolinon | 0,05 | 0,05 | - | - |
| Phenoxyethanol | 0,50 | 0,50 | 0,40 | 0,40 |
| Piroctone Olamine | - | - | - | 0,20 |
| Nylon-12 (1,8-Diazacyclotetradecan-2,7-dion Homopolymer) | 0,50 | - | 0,50 | 0,50 |
| Distärkephopsphat | - | 1,00 | - | 0,50 |
| Methyl Methacrylate Crosspolymer | - | 0,50 | 0,50 | - |
| Caprylyl Glycol | - | - | 0,30 | - |
| 1,2-Hexandiol | - | - | - | 0,50 |
| Butylenglykol | - | - | 2,00 | 2,00 |
| DMDM Hydantoin | - | - | 0,15 | - |
| Glycyrrhiza Inflata Root Extrakt (Süßholzwurzel) | - | - | 0,05 | - |
| N-(4-(2,4-dihydroxyphenyl)thiazol-2-yl)-4-(hydroxymethyl)benzamid | 0,10 | 0,10 | 0,05 | 0,05 |
| N-(4-(2,4-dihydroxyphenyl)thiazol-2-yl)-4-(2-hydroxypropan-2-yl)benzamid | 0,10 | 0,25 | 0,15 | 0,10 |
| N-(4-(2,4-dihydroxyphenyl)thiazol-2-yl)-6-(hydroxymethyl)nicotinamid | 0,15 | 0,15 | 0,01 | 0,06 |
| N-(4-(2,4-dihydroxyphenyl)thiazol-2-yl)benzamid | 0,20 | 0,30 | 0,10 | 0,05 |
| Hydroxyisohexyl 3-Cyclohexencarboxaldehyd | 0,05 | 0,05 | 0,05 | - |
| Citronellol | - | 0,05 | - | 0,05 |
| Benzylsalicylat | - | - | 0,05 | 0,05 |
| Parfüm | 0,20 | 0,20 | 0,20 | 0,20 |
| Natriumhydroxid | q.s. | q.s. | q.s. | q.s. |
| Wasser | ad 100 | ad 100 | ad 100 | ad 100 |

| **Rezepturbeispiele** | **49** | **50** | **51** | **52** |
|---|---|---|---|---|
| **Chemische/INCI-Bezeichnung** | **Gew.-%** | **Gew.-%** | **Gew.-%** | **Gew.-%** |
| Kalium Cetylphosphat | 0,20 | 0,20 | 0,25 | 0,20 |
| C12-15 Alkyl Benzoat | 2,50 | 2,50 | 2,00 | 2,00 |
| Isopropyl Palmitat | 2,50 | 2,50 | - | 3,00 |
| Isopropy Stearat | - | - | 2,00 | - |
| Caprylsäure/Caprinsäure Triglycerid | 2,50 | 2,50 | 1,50 | 2,00 |
| Glyceryl Stearat | 1,00 | 1,00 | 1,25 | 1,50 |
| Octyldodecanol | - | - | 1,50 | - |
| Paraffinum Liquidum (Mineralöl) | - | - | - | 1,00 |
| Glycerin | 5,00 | 7,00 | 9,00 | 6,00 |
| Bis-Ethylhexyloxyphenol Methoxyphenyl Triazines | - | 1,00 | - | 1,00 |
| Titandioxid + Trimethoxycaprylylsilan | - | - | 1,00 | 1,00 |
| Phenylbenzimidazole Sulfonsäure | - | - | 1,00 | 1,00 |
| Butyl Methoxydibenzoylmethan | 1,00 | - | 2,00 | 2,00 |
| Dinatrium Phenyl Dibenzimidazol Tetrasulfonat | - | 1,50 | 1,00 | - |
| Ethylhexyltriazon | 1,00 | - | - | - |
| Ethylhexyl Methoxycinnamat + BHT | 2,00 | - | - | - |
| Carbomer | | 0,15 | 0,20 | 0,30 |
| Acrylates/C10-30 Alkyl Acrylate Crosspolymer | 0,30 | 0,10 | 0,15 | - |
| Xanthan Gummi | | | 0,15 | 0,10 |
| Methylisothiazolinon | 0,05 | - | - | - |
| Phenoxyethanol | 0,50 | 0,50 | 0,40 | 0,40 |
| Methylparaben | - | 0,10 | - | - |
| Ethylhexylsalicylat | - | - | 0,30 | - |
| Butylenglycol | - | - | 3,00 | 3,00 |
| Benzethonium chlorid | - | - | - | 0,10 |
| N-(4-(2,4-dihydroxyphenyl)thiazol-2-yl)-6-(hydroxymethyl)nicotinamid | 0,10 | 0,10 | 0,05 | 0,05 |
| N-(4-(2,4-dihydroxyphenyl)thiazol-2-yl)benzamid | 0,10 | 0,25 | 0,15 | 0,10 |
| 4-cyano-N-(4-(2,4-dihydroxyphenyl)thiazol-2-yl)benzamid | 0,15 | 0,15 | 0,01 | 0,06 |
| 4-acetyl-N-(4-(2,4-dihydroxyphenyl)thiazol-2-yl)benzamid | 0,20 | 0,30 | 0,10 | 0,05 |
| Coumarin | - | 0,05 | - | 0,05 |
| Linalool | 0,05 | - | - | 0,05 |
| Hexylcinnamal | 0,05 | 0,05 | - | - |
| 1-(1,2,3,4,5,6,7,8-octahydro-2,3,8,8,-tetramethyl-2-naphthyl)ethan-1-on | - | - | 0,10 | - |
| Parfüm | 0,10 | 0,30 | 0,20 | 0,30 |
| BHT (tert-Butylhydroxytoluol) | 0,05 | - | - | - |
| Tocopheryl Acetat | - | 0,10 | - | - |
| Natriumhydroxid | q.s. | q.s. | q.s. | q.s. |
| Wasser | ad 100 | ad 100 | ad 100 | ad 100 |

### W/O Emulsion

| **Rezepturbeispiele** | **53** | **54** |
|---|---|---|
| **Chemische/INCI-Bezeichnung** | **Gew**.**-%** | **Gew.-%** |
| Polygylceryl-3 Düsostearat | 1,5 | 1,5 |
| PEG-40 Sorbitan Perisostearat | 2,5 | 2,5 |
| Lanolin Alkohol | 0,5 | 0,5 |
| Paraffinum Liquidum (Mineralöl) | 8 | 8 |
| Cera Microcrystallina | 2,5 | 2,5 |
| Cyclomethicon | 4 | 4 |
| Isohexadecan | 2 | 2 |
| Isopropylpalmitat | 5 | 5 |
| lodopropynyl Butylcarbamat | - | 0,1 |
| Magnesiumsulfat | 0,5 | 0,5 |
| Kaliumsorbat | 0,1 | - |
| Benzylsalicylat | 0,1 | - |
| N-(4-(2,4-dihydroxyphenyl)thiazol-2-yl)-4-(hydroxymethyl)benzamid | 0.10 | 0.05 |
| N-(4-(2,4-dihydroxyphenyl)thiazol-2-yl)-6-(hydroxymethyl)nicotinamid | 0.25 | 0.15 |
| N-(4-(2,4-dihydroxyphenyl)thiazol-2-yl)-4-(hydroxymethyl)benzamid | 0.15 | 0.30 |
| N-(4-(2,4-dihydroxyphenyl)thiazol-2-yl)benzo[d][1,3]dioxole-5-carboxamid | 0.10 | 0.15 |
| N-(4-(2,4-dihydroxyphenyl)thiazol-2-yl)picolinamid | 0.10 | 0.05 |
| 4-(tert-butyl)-N-(4-(2,4-dihydroxyphenyl)thiazol-2-yl)benzamid | 0.25 | 0.15 |
| Glycerin | 7 | 7 |
| Parfüm | q.s. | q.s. |
| Wasser | ad 100 | ad 100 |

### Deo/AT-Beispielrezepturen

| **Rezepturbeispiele** | **55** | **56** | **57** | **58** |
|---|---|---|---|---|
| **Chemische/INCI**-**Bezeichnung** | **Gew.- %** | **Gew.- %** | **Gew.- %** | **Gew.- %** |
| Polyethylenglykol(21)stearylether | 2,50 | 2,50 | 1,50 | 1,50 |
| Polyethylenglykol(2)stearylether | 1,50 | 1,50 | 2,50 | 2,50 |
| Polypropylenglykol(15)stearylether | 3,00 | 3,00 | 4,00 | 4,00 |
| Trinatriumsalz der Ethylendiamintetraessigsäure (20% wäßr. Lösung) | 1,50 | 1,50 | 1,50 | 1,50 |
| Persea Gratissima Öl (Avokadoöl) | 0,10 | 0,10 | 0,15 | 0,15 |
| Parfüm | q.s. | q.s. | q.s. | q.s. |
| 1-(1,2,3,4,5,6,7,8-octahydro-2,3,8,8,-tetramethyl-2-naphthyl)ethan-1-one | 0,10 | 0,05 | - | 0,05 |
| Linaylacetat | - | 0,05 | 0,05 | - |
| Citronellol | - | - | 0,05 | - |
| Triethylcitrat | - | - | - | 0,05 |
| N-(4-(2,4-dihydroxyphenyl)thiazol-2-yl)-4-(hydroxymethyl)benzamid | 0,10 | 0,10 | 0,05 | 0,05 |
| N-(4-(2,4-dihydroxyphenyl)thiazol-2-yl)-4-(2-hydroxypropan-2-yl)benzamid | 0,10 | 0,25 | 0,15 | 0,10 |
| N-(4-(2,4-dihydroxyphenyl)thiazol-2-yl)-6-(hydroxymethyl)nicotinamid | 0,15 | 0,15 | 0,01 | 0,06 |
| N-(4-(2,4-dihydroxyphenyl)thiazol-2-yl)benzamid | 0,20 | 0,30 | 0,10 | 0,05 |
| Wasser, ad | ad 100 | ad 100 | ad 100 | ad 100 |

| **Rezepturbeispiele** | **59** | **60** | **61** | **62** |
|---|---|---|---|---|
| **Chemische/INCI**-**Bezeichnung** | **Gew.-%** | **Gew.-%** | **Gew.-%** | **Gew.-%** |
| Isoceteth-20 | 3,50 | 3,00 | 4,00 | 4,00 |
| Glycerylisostearat | 2,00 | 2,00 | 2,00 | 2,50 |
| Dicaprylylether | - | 0,50 | 2,00 | 2,50 |
| Capryl-Caprinsäureester | 2,00 | 1,50 | - | - |
| Aluminiumchlorhydrat | 5,00 | 5,00 | - | 3,00 |
| Persea Gratissima Öl (Avokadoöl) | - | - | 0,20 | - |
| Polyethylenglycol(150)distearat | 0,50 | 0,50 | 1,00 | 1,00 |
| Glycerin | 4,00 | 2,00 | - | 2,00 |
| Butylenglykol | - | 3,00 | 1,00 | 2,00 |
| Propylenglykol | 3,00 | - | 3,00 | - |
| 4-[(Cyclopentylhydroxyphenylacetyl)oxy]-1,1-dimethyl-piperidiniumbromid | 0,05 | 0,10 | - | - |
| N-(4-(2,4-dihydroxyphenyl)thiazol-2-yl)-4-(hydroxymethyl)benzamid | 0,10 | 0,01 | 0,20 | 0,25 |
| N-(4-(2,4-dihydroxyphenyl)thiazol-2-yl)-6-(hydroxymethyl)nicotinamid | 0,30 | 0,30 | 0,01 | 0,06 |
| 4-cyano-N-(4-(2,4-dihydroxyphenyl)thiazol-2-yl)benzamid | 0,02 | 0,09 | 0,25 | 0,15 |
| N-(4-(2,4-dihydroxyphenyl)thiazol-2-yl)benzo[d][1,3]dioxole-5-carboxamid | 0,31 | 0,25 | 0,20 | 0,02 |
| Geraniol | - | 0,05 | - | - |
| Ethyllinalool | - | - | 0,05 | |
| Linalool | - | - | - | 0,10 |
| Parfüm | 0,25 | 0,50 | 0,50 | 0,75 |
| Wasser, ad | ad 100 | ad 100 | ad 100 | ad 100 |

| **Rezepturbeispiele** | **63** | **64** | **65** | **66** |
|---|---|---|---|---|
| **Chemische/INCI**-**Bezeichnung** | **Gew.-%** | **Gew.-%** | **Gew.-%** | **Gew.-%** |
| Polyoxyethylen(20)cetylstearylether | 3,00 | 3,00 | 4,00 | 4,00 |
| Polyoxyethylen(12)cetylstearylether | 0,50 | 0,50 | - | - |
| Glycerinstearat | 3,00 | 3,00 | 3,00 | 3,00 |
| Cetylstearylalkohol | 0,50 | 0,50 | - | - |
| Cetylpalmitat | 0,50 | 0,50 | - | - |
| Capryl-Caprinsäureester | 4,00 | 4,00 | 3,50 | 3,50 |
| Di-n-Octylether | 5,00 | 5,00 | 5,00 | 5,00 |
| Polyethylenglycol(150)distearat | - | - | 1,00 | 1,00 |
| Glycerin | 4,00 | 4,00 | 2,00 | 2,00 |
| N-(4-(2,4-dihydroxyphenyl)thiazol-2-yl)-4-(hydroxymethyl)benzamid | 0,10 | 0,10 | 0,05 | 0,05 |
| N-(4-(2,4-dihydroxyphenyl)thiazol-2-yl)benzamid | 0,10 | 0,25 | 0,15 | 0,10 |
| N-(4-(2,4-dihydroxyphenyl)thiazol-2-yl)-4-(2-hydroxypropan-2-yl)benzamid | 0,15 | 0,15 | 0,01 | 0,06 |
| 4-(tert-butyl)-N-(4-(2,4-dihydroxyphenyl)thiazol-2-yl)benzamid | 0,20 | 0,30 | 0,10 | 0,05 |
| Hexylcinnamal | 0,05 | 0,10 | | |
| 1-(1,2,3,4,5,6,7,8-octahydro-2,3,8,8,-tetramethyl-2-naphthyl)ethan-1-one | | 0,05 | 0,10 | |
| 3-Methyl-5-phenyl-1-pentanol | 0,05 | | | 0,05 |
| Parfüm | 0,30 | 0,30 | 0,50 | 0,50 |
| Wasser, ad | ad 100,00 | ad 100,00 | ad 100,00 | ad 100,00 |

| **Rezepturbeispiele** | **67** | **68** | **69** | **70** |
|---|---|---|---|---|
| **Chemische/INCI**-**Bezeichnung** | **Gew.-%** | **Gew.-%** | **Gew.-%** | **Gew.-%** |
| Steareth-100 | 1,00 | 1,00 | 1,00 | 1,00 |
| Polyglyceryl-3 Diisostearat | 1,60 | 1,60 | 1,60 | 1,60 |
| PEG-45/Dodecyl Glycol Copolymer | 0,80 | 0,80 | 0,80 | 0,80 |
| C20-40 Alkyl Stearat | 10,00 | 10,00 | 10,00 | 10,00 |
| Caprylic/Capric Triglycerid | 3,00 | 3,00 | 3,00 | 3,00 |
| Octyldodecanol | 3,00 | 3,00 | 3,00 | 3,00 |
| Dicaprylyl Ether | 4,00 | 4,00 | 4,00 | 4,00 |
| Butylenglykol | 4,00 | 4,00 | 4,00 | 4,00 |
| N-(4-(2,4-dihydroxyphenyl)thiazol-2-yl)-4-(2-hydroxypropan-2-yl)benzamid | 0,01 | 0,10 | 0,15 | 0,30 |
| N-(4-(2,4-dihydroxyphenyl)thiazol-2-yl)benzamid | 0,20 | 0,30 | 0,10 | 0,25 |
| N-(4-(2,4-dihydroxyphenyl)thiazol-2-yl)-6-(hydroxymethyl)nicotinamid | 0,01 | 0,10 | 0,20 | 0,35 |
| N-(4-(2,4-dihydroxyphenyl)thiazol-2-yl)benzamid | 0,02 | 0,30 | 0,25 | 0,15 |
| Hydroxyisohexyl 3-Cyclohexencarboxaldehyd | 0,05 | 0,05 | 0,05 | 0,05 |
| Parfüm | 0,35 | 0,30 | 0,25 | 0,15 |
| Wasser, ad | ad 100,00 | ad 100,00 | ad 100,00 | ad 100,00 |

### Beispielrezepturen

| **Rezepturbeispiele** | **71** | **72** | **73** | **74** |
|---|---|---|---|---|
| **Chemische/INCI-Bezeichnung** | **Gew.-%** | **Gew.-%** | **Gew.-%** | **Gew.-%** |
| Alkohol denat. | 20,0 | 20,0 | 30,0 | 30,0 |
| Hydroxyethylcellulose | 0,40 | 0,40 | 0,30 | 0,30 |
| Polyethylenglykol 400 | 3,00 | 3,00 | 2,00 | 2,00 |
| Polyethylenglykol (2000) hydriertes Rizinusöl | 2,00 | 2,00 | 3,00 | 3,00 |
| Persea Gratissima Öl (Avokadoöl) | 0,50 | 0,50 | 0,10 | 0,10 |
| 4-[(Cyclopentylhydroxyphenylacetyl)oxy]-1,1-dimethyl-piperidiniumbromid | 0,10 | 0,30 | - | - |
| N-(4-(2,4-dihydroxyphenyl)thiazol-2-yl)-4-(hydroxymethyl)benzamid | 0,05 | 0,05 | 0,30 | 0,30 |
| 4-cyano-N-(4-(2,4-dihydroxyphenyl)thiazol-2-yl)benzamid | 0,10 | 0,25 | 0,15 | 0,10 |
| N-(4-(2,4-dihydroxyphenyl)thiazol-2-yl)-4-(2-hydroxypropan-2-yl)benzamid | 0,15 | 0,15 | 0,01 | 0,06 |
| Coumarin | - | - | 0,05 | - |
| Benzylsalicylat | - | 0,05 | - | - |
| Butylphenylmethylpropional | 0,05 | - | - | - |
| Parfüm | 0,25 | 0,30 | 0,50 | 0,30 |
| Wasser, ad | ad 100 | ad 100 | ad 100 | ad 100 |

| **Rezepturbeispiele** | **75** | **76** | **77** | **78** |
|---|---|---|---|---|
| **Chemische/INCI-Bezeichnung** | **Gew.%** | **Gew.%** | **Gew.-%** | **Gew.-%** |
| 2-Octyldodecanol | 0,50 | 0,50 | 0,50 | 0,50 |
| 1,2-Propylenglykol | 1,00 | 1,00 | 1,00 | 1,00 |
| 2-Butyloctansäure | 0,25 | - | 0,25 | - |
| Aluminiumchlorhydrat | 2,00 | 3,00 | - | 3,00 |
| 4-cyano-N-(4-(2,4-dihydroxyphenyl)thiazol-2-yl)benzamid | 0,01 | 0,10 | 0,15 | 0,30 |
| N-(4-(2,4-dihydroxyphenyl)thiazol-2-yl)-4-(hydroxymethyl)benzamid | 0,20 | 0,30 | 0,10 | 0,25 |
| N-(4-(2,4-dihydroxyphenyl)thiazol-2-yl)benzamid | 0,01 | 0,10 | 0,20 | 0,35 |
| N-(4-(2,4-dihydroxyphenyl)thiazol-2-yl)-4-(2-hydroxypropan-2-yl)benzamid | 0,02 | 0,30 | 0,25 | 0,15 |
| Linalool | 0,05 | - | 0,05 | 0,05 |
| Coumarin | - | - | 0,05 | - |
| Benzylsalicylat | 0,05 | 0,05 | - | 0,05 |
| Parfüm | 0,10 | 0,20 | 0,40 | 0,20 |
| Ethanol | ad 100 | ad 100 | ad 100 | ad 100 |

Die durch Zusammenmischung der jeweiligen Bestandteile erhaltene flüssige Phase wird mit einem Propan-Butan-Gemisch (2.7) im Verhältnis 39:61 in Aerosolbehälter abgefüllt.

| **Rezepturbeispiele** | **79** | **80** | **81** |
|---|---|---|---|
| **Chemische Bezeichnung** | Gew.- % | Gew.- % | Gew.- % |
| Alkohol denat. | 20,0 | 30,0 | 20,0 |
| Hydroxyethylcellulose | 0,40 | 0,30 | 0,40 |
| Polyethylenglykol 400 | 3,00 | 2,00 | 3,00 |
| Polyethylenglykol (2000) hydriertes Rizinusöl | 2,00 | 3,00 | 2,00 |
| Persea Gratissima Öl (Avokadoöl) | 0,50 | 0,10 | 0,50 |
| 4-[(Cyclopentylhydroxyphenylacetyl)oxy]-1,1-dimethyl-piperidiniumbromid | 0,05 | - | - |
| N-(4-(2,4-dihydroxyphenyl)thiazol-2-yl)-4-(2-hydroxypropan-2-yl)benzamid | 0,05 | 0,05 | 0,30 |
| N-(4-(2,4-dihydroxyphenyl)thiazol-2-yl)-6-(hydroxymethyl)nicotinamid | 0,10 | 0,25 | 0,15 |
| N-(4-(2,4-dihydroxyphenyl)thiazol-2-yl)benzamid | 0,15 | 0,15 | 0,01 |
| 2-Butyloctansäure | - | 0,10 | - |
| Geraniol | - | 0,05 | - |
| Citronellol | 0,05 | - | - |
| Ethyllinalool | - | - | 0,05 |
| Parfüm | 0,30 | 0,40 | 0,20 |
| Wasser, ad | ad 100 | ad 100 | ad 100 |

| **Rezepturbeispiele** | **82** | **83** | **84** |
|---|---|---|---|
| **Chemische/INCI-Bezeichnung** | **Gew.%** | **Gew.%** | **Gew.%** |
| Glycerinmonostearat | 5,00 | 5,00 | 5,00 |
| Polyethylenglykol(2000) monostearat | 2,00 | 2,00 | 2,00 |
| Stearylalkohol | 3,00 | 3,00 | 3,00 |
| Cyclometicon | 4,00 | 4,00 | 4,00 |
| Paraffinöl | 6,00 | 6,00 | 6,00 |
| Trinatrium EDTA | 0,20 | 0,20 | 0,20 |
| Aluminiumchlorhydrat | 2,50 | 2,50 | 2,50 |
| N-(4-(2,4-dihydroxyphenyl)thiazol-2-yl)-4-(hydroxvmethyl)benzamid | 0,10 | 0,25 | 0,15 |
| N-(4-(2,4-dihydroxyphenyl)thiazol-2-yl)benzamid | 0,15 | 0,15 | 0,01 |
| 4-cyano-N-(4-(2,4-dihydroxyphenyl)thiazol-2-yl)benzamid | 0,10 | 0,25 | 0,15 |
| 2-Methylpropandiol | 3,00 | 3,00 | 3,00 |
| 2-Ethylhexylglycerinether | 0,50 | 0,50 | 0,50 |
| Benzylsalicylat | - | - | 0,05 |
| Triethylcitrat | - | 0,05 | 0,05 |
| Hexalcinnamal | 0,05 | - | - |
| Parfüm | 0,40 | 0,30 | 0,20 |
| Wasser, ad | 100 | 100 | 100 |

## Patentansprüche

1. Aromatische Amidothiazole, **dadurch gekennzeichnet, daß** sie eine der folgenden Strukturen aufweisen: 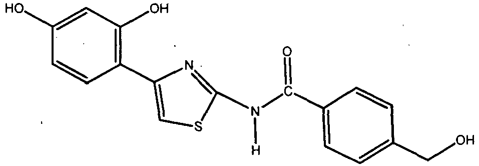 N-(4-(2,4-dihydroxyphenyl)thiazol-2-yl)-4-(hydroxymethyl)benzamid 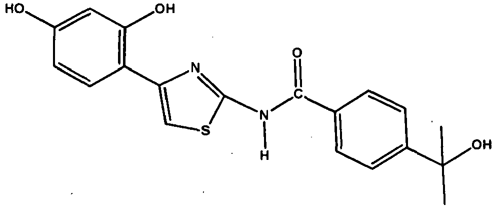 N-(4-(2,4-dihydroxyphenyl)thiazol-2-yl)-4-(2-hydroxypropan-2-yl)benzamid 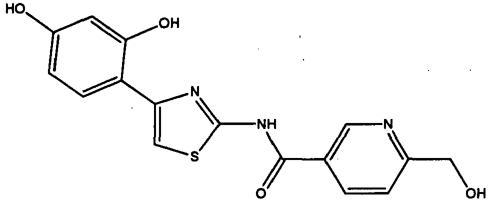 *N*-(4-(2,4-dihydroxyphenyl)thiazol-2-yl)-6-(hydroxymethyl)nicotinamide 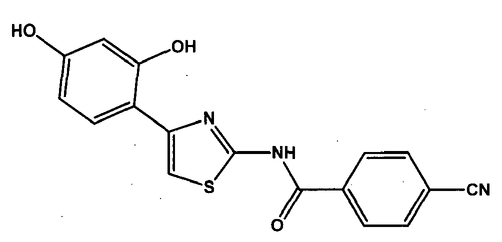 4-cyano-*N*-(4-(2,4-dihydroxyphenyl)thiazol-2-yl)benzamide 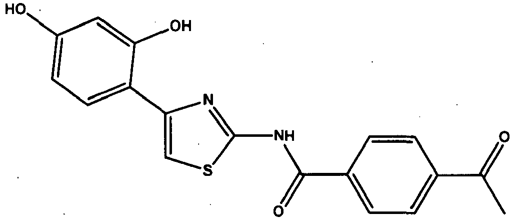 4-acetyl-*N*-(4-(2,4-dihydroxyphenyl)thiazol-2-yl)benzamide 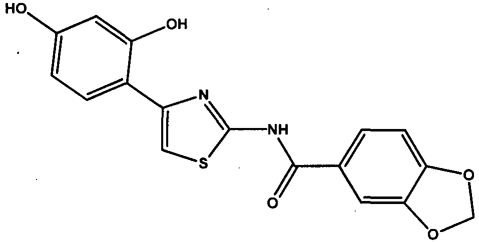 *N*-(4-(2,4-dihydroxyphenyl)thiazol-2-yl)benzo[*d*][1,3]dioxole-5-carboxamide 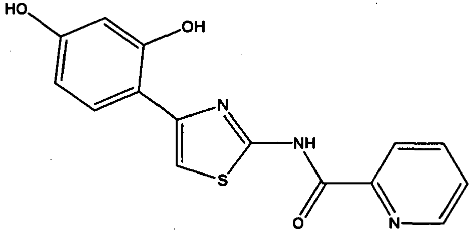 *N*-(4-(2,4-dihydroxyphenyl)thiazol-2-yl)picolinamide 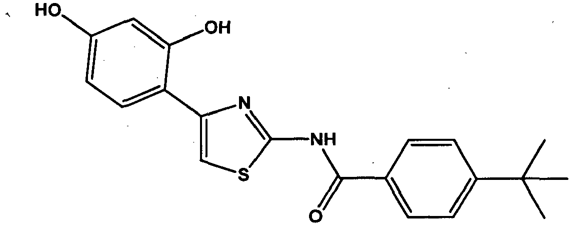 4-(*tert*-butyl)-*N*-(4-(2,4-dihydroxyphenyl)thiazol-2-yl)benzamide 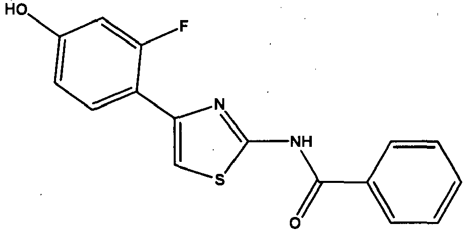 *N*-(4-(2-fluoro-4-hydroxyphenyl)thiazol-2-yl)benzamide

2. Amidothiazole nach Anspruch 1, **dadurch gekennzeichnet, daß** sie als Halogenid, Carbonat, Ascorbat, , Sulfat, Acetat und/oder Phosphat vorliegen.

3. Kosmetische oder dermatologische Zubereitungen mit einem Gehalt an einem oder mehreren aromatischen Amidothiazolen, wie sie in Anspruch 1 oder 2 definiert sind.

4. Zubereitungen nach Anspruch 3, enthaltend 0,000001 bis 10 Gew.-%, insbesondere 0,0001 bis 3 Gew.-%, ganz besonders 0,001 bis 1 Gew.-% an einem oder mehreren der in einem der Anspruch 1 oder 2 definierten aromatischen Amidothiazole, bezogen auf das Gesamtgewicht der Zubereitung.

5. Verwendung eines oder mehrerer in Anspruch 1 oder 2 definierten aromatischen Amidothiazole, oder Zubereitungen, eines oder mehrere solcher aromatischer Amidothiazole enthaltend, zur kosmetischen Behandlung und/oder Prophylaxe unerwünschter Hautpigmentierung.

6. In Anspruch 1 oder 2 definierte aromatischen Amidothiazole zur kosmetischen oder dermatologischen Behandlung und/oder Prophylaxe unerwünschter Hautpigmentierung.

## Claims

1. Aromatic amidothiazoles, **characterized in that** they have one of the following structures: 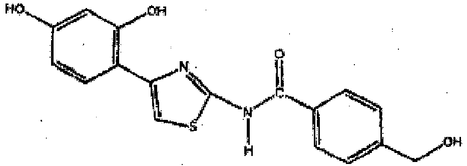 N-(4-(2,4-dihydroxyphenyl)thiazol-2-yl)-4-(hydroxymethyl)benzamide 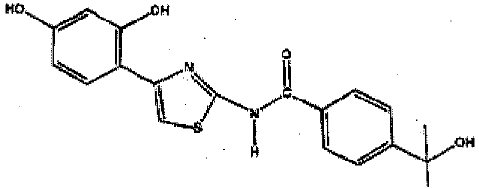 -(4-(2,4-dihydroxyphenyl)thiazol-2-yl)-4-(hydroxypropan-2-yl)benzamide 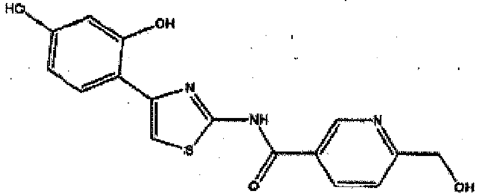 *N*-(4-(2,4-dihydroxyphenyl)thiazol-2-yl)-6-(hydroxymethyl)nicotinamide 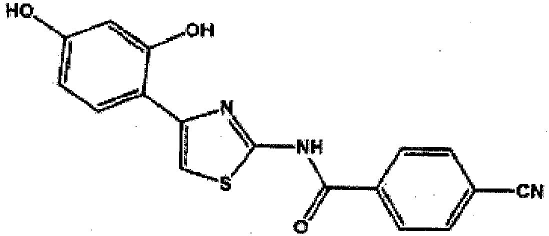 4-cyano-*N-*(4-(2,4-dihydroxyphenyl)thiazol-2-yl)benzamide 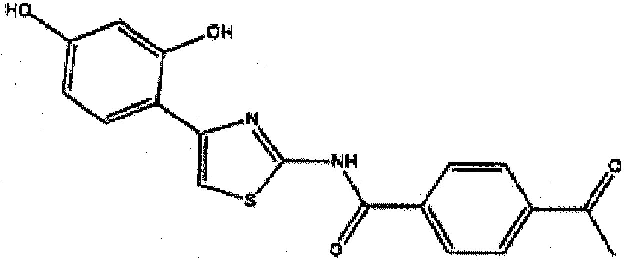 4-acetyl-*N*-(4-(2,4-dihydroxyphenyl)thiazol-2-yl)benzamide 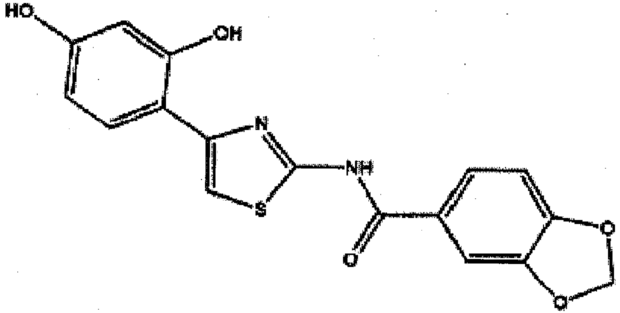 *N*-(4-(2,4-dihydroxyphenyl)thiazol-2-yl)benzo[*d*]dioxole-5-carboxamide 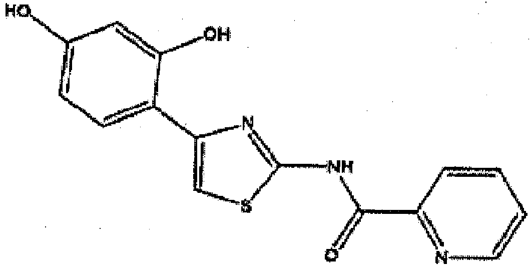 *N*-(4-(2,4-dihydroxyphenyl)thiazol-2-yl)plcolinamide 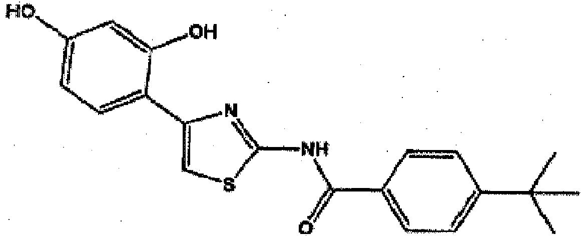 4-(*tert-*butyl)-*N-*(4-(2,4-dihydroxyphenyl)thiazol-2-yl)benzamide 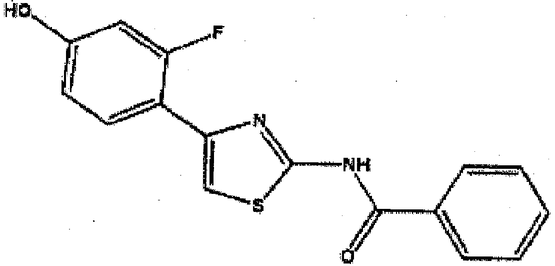 *N*-(4-(2-fluoro-4-hydroxyphenyl)thiazol-2-yl)benzamide

2. Amidothiazoles according to Claim 1, **characterized in that** they are present as halide, carbonate, ascorbate, sulphate, acetate and/or phosphate.

3. Cosmetic or dermatological preparations with a content of one or more aromatic amidothiazoles, as defined in Claim 1 or 2.

4. Preparations according to Claim 3, comprising 0.000001 to 10% by weight, in particular 0.0001 to 3% by weight, very particularly 0.001 to 1% by weight, of one or more of the aromatic amidothiazoles defined in one of Claims 1 and 2, based on the total weight of the preparation.

5. Use of one or more aromatic amidothiazoles defined in Claim 1 or 2, or preparations comprising one or more of such aromatic amidothiazoles for the cosmetic treatment and/or prophylaxis of undesired skin pigmentation.

6. Aromatic amidothiazoles defined in Claim 1 or 2 for the cosmetic or dermatological treatment and/or prophylaxis of undesired skin pigmentation.

## Revendications

1. Amidothiazoles aromatiques, **caractérisés en ce qu'**ils présentent une des structures suivantes 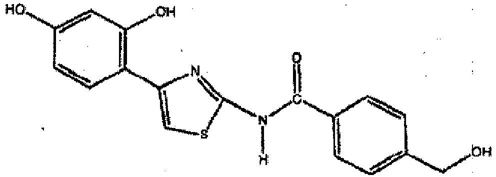 N-(4-(2,4-dihydroxyphényl)thiazol-2-yl)-4-(hydroxyméthyl)benzamide 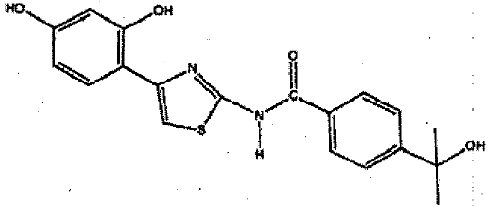 N-(4-(2,4-dihydroxyphényl)thiazol-2-yl)-4-(2-hydroxypropan-2-yl)benzamide 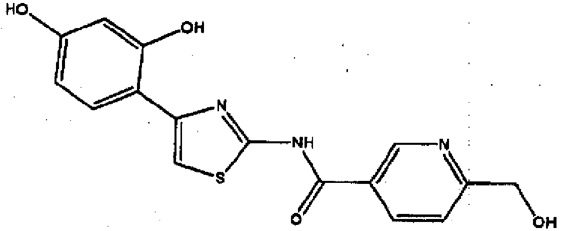 N-(4-(2,4-dihydroxyphényl)thrazol-2-yl)-6-(hydroxyméthyl)nicotinamide 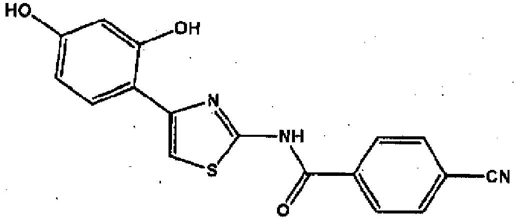 4-cyano-N-(4-(2,4-dihydroxyphényl)thiazol-2-yl)benzamide 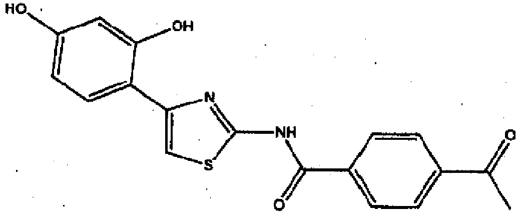 4-acétyl-N-(4-(2,4-dihydroxyphényl)thiazol-2-yl)benzamide 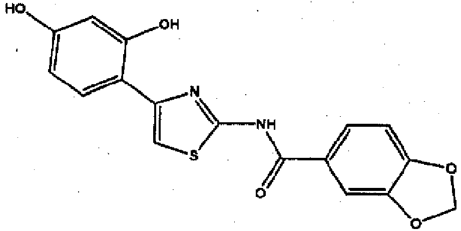 N-(4-(2,4-dihydroxyphényl)thiazol-2-yl)benzo[d][1,3]dioxole-5-carboxamide 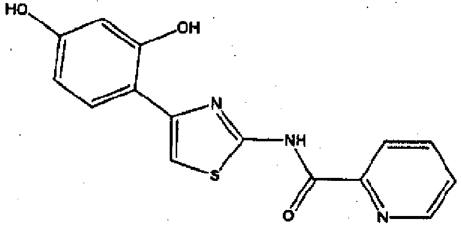 N-(4-(2,4-dihydroxyphényl)thiazol-2-yl)picolinamide 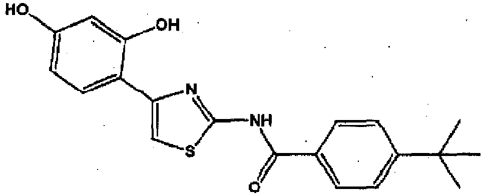 4-(tert-butyl)-N-(4-(2,4-dihydroxyphényl)thiazol-2-yl)benzamide 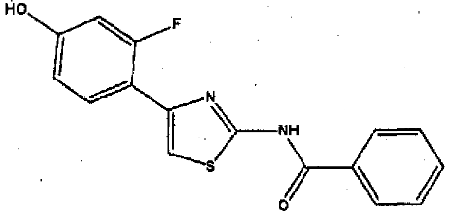 N-(4-(2-fluoro-4-hydroxyphényl)thiazol-2-yl)benzamide.

2. Amidothiazoles selon la revendication 1, **caractérisés en ce qu'**ils sont présentent sous la forme d'un halogénure, d'un carbonate, d'un ascorbate, d'un sulfate, d'un acétate et/ou d'un phosphate.

3. Préparations cosmétiques ou dermatologiques ayant une teneur en un ou plusieurs amidothiazoles aromatiques tels que définis dans la revendication 1 ou 2.

4. Préparations selon la revendication 3, contenant 0,000001 à 10 % en poids, notamment 0,0001 à 3 % en poids, tout particulièrement 0,001 à 1 % en poids, d'un ou de plusieurs des amidothiazoles aromatiques définis dans l'une quelconque des revendications 1 ou 2, par rapport au poids total de la préparation.

5. Utilisation d'un ou de plusieurs amidothiazoles aromatiques définis dans la revendication 1 ou 2, ou de préparation contenant un ou plusieurs tels amidothiazoles aromatiques, pour le traitement cosmétique et/ou la prophylaxie d'une pigmentation cutanée indésirable.

6. Amidothiazoles aromatiques définis dans la revendication 1 ou 2 pour le traitement cosmétique ou dermatologique et/ou la prophylaxie d'une pigmentation cutanée indésirable.
